(19) Europäisches Patentamt
European Patent Office
Office européen des brevets

(11) Publication number: **0 138 552**
**A2**

# (12) EUROPEAN PATENT APPLICATION

(21) Application number: **84306866.9**

(22) Date of filing: **09.10.84**

(51) Int. Cl.⁴: **C 07 D 501/46**
// C07D471/04, C07D498/04, C07D513/04

(30) Priority: **17.10.83 US 542619**

(43) Date of publication of application: **24.04.85**
**Bulletin 85/17**

(84) Designated Contracting States: **AT BE CH DE FR GB IT LI LU NL SE**

(71) Applicant: **ELI LILLY AND COMPANY, 307, East McCarty Street, Indianapolis Indiana 46285 (US)**

(72) Inventor: **Katner, Allen Samuel, 219 W. 81st Street, Indianapolis Indiana 46260 (US)**

(74) Representative: **Hudson, Christopher Mark et al, Erl Wood Manor, Windlesham Surrey GU20 6PH (GB)**

## (54) Improvements on or relating to 3-bicyclicpyridinium-methyl cephalosporins.

(57) Cephalosporin compounds substituted in the 7-position by a 2-(5- or 6-membered heterocyclic)-2-oximinoacetylamino group and of the formula

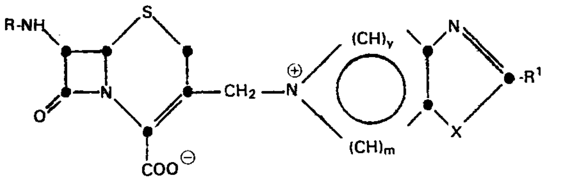 1

in which R is hydrogen, formyl, α-aminoadipoyl, protected α-aminoadipoyl, or an acyl group of the formula

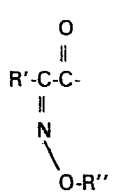

in which R' is a 5- or 6-membered heterocyclic ring of the formulae

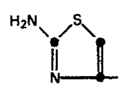 , 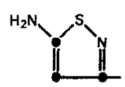 , 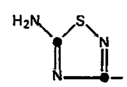 , 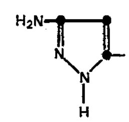 ,

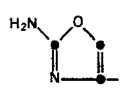 , 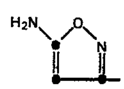 , 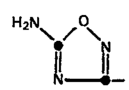 , 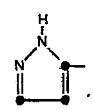 ,

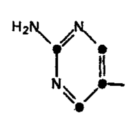 , 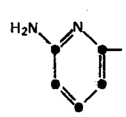 , or 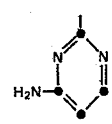 ;

R'' is hydrogen, $C_1$-$C_4$ alkyl, a carboxy-substituted alkyl or carboxy-substituted cycloalkyl group of the formula:

a
|
$-C-(CH_2)_n-COR'''$
|
b

EP 0 138 552 A2

in which n is 0-3; a and b when taken separately are, independently, hydrogen or $C_1$-$C_3$ alkyl, and when taken together with the carbon to which they are bonded form a $C_3$-$C_7$ carbocyclic ring; R''' is hydrogen, $C_1$-$C_4$ alkoxy, amino, or OR°, in which R° is indanyl, phthalidyl, or an acyloxymethyl group of the formula $-CH_2-O-C(O)-R_2$ in wich $R_2$ is $C_1$-$C_4$ alkyl or phenyl; or COOR° is a protected carboxy group;
or R'' is an N-substituted carbamoyl group of the formula

O
||
-C-NHR''''

in which R'''' is $C_1$-$C_4$ alkyl, phenyl or $C_1$-$C_3$ alkyl substituted by phenyl;
Y and m, independently, are integers equal to 0, 1, 2 or 3, provided that y plus m equals 3;
$R^1$ is hydrogen, $C_1$-$C_4$ alkyl, phenyl, thienyl, amino or $C_1$-$C_4$ alkanoylamino;
X is O, S or N-$R^2$, where $R^2$ is hydrogen or $C_1$-$C_4$ alkyl; or a pharmaceutically-acceptable salt or biologically-cleavable ester thereof.

Title

IMPROVEMENTS ON OR RELATING TO
3-BICYCLICPYRIDINIUM-METHYL CEPHALOSPORINS

This invention relates to novel cephalosporin antibiotics which structurally contain a 7-[2-(amino-substituted-5- or 6-membered heterocyclic ring)-2-oxy-iminoacetylamino] side chain and a bicyclic pyridinium methyl group in the 3-position of the cephalosporin nucleus.

Prior to the present invention, a number of cephalosporin antibiotics substituted in the 3-position by a quaternary ammonium methyl and in the 7-position with various acylamino groups were known. Such compounds possess the zwitterionic structure in that the positively-charged nitrogen atom of the quaternary ammonium group exists in the salt form with the anionic form of the C-4 carboxy group (carboxylate anion) of the cephalosporin. The well-known cephalosporin antibiotic cephaloridine, 7-(α-thienylacetamido)-3-(pyridinium-1-ylmethyl)-3-cephem-4-carboxylate of the following formula, possesses the zwitterionic structure:

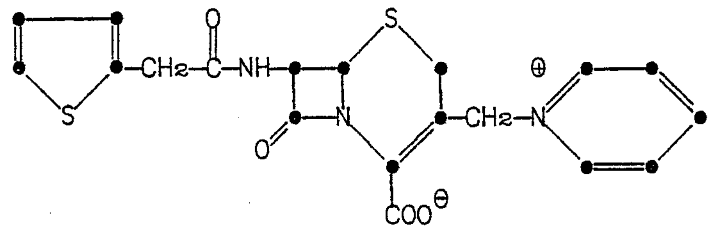

The first cephalosporin of this type was discovered by Hale, Newton, and Abraham, Biochem. J. 79, 403 (1961), upon the reaction of cephalosporin C with pyridine (cephalosporin $C_A$). Numerous other cephalosporins of this type with differing 7-acylamino side chains have been described since cephalosporin $C_A$ and cephaloridine were discovered.

Recently, Heymes et al., U.S. Patent No. 4,152,432, described cephalosporin antibiotics having as the 7-acylamino side chain a 7-[2-(2-aminothiazol-4-yl)-2-alkoxyiminoacetylamino] group and as the 3-position substituent an acetoxymethyl group. Others have prepared zwitterionic derivatives of this antibiotic, e.g., as described in U.S. Patent No. 4,098,888, by Takeda and in U.S. Patent No. 4,258,041, by O'Callagan et al.

Because the cephalosporin antibiotics possess potent antibacterial activity, intensive research to find improved broad spectrum cephalosporin antibiotics continues. In particular, these efforts seek improved cephalosporin antibiotics having potent broad spectrum activity coupled with activity against bacteria and bacterial strains known to be resistant to antibiotics

in current use. This invention provides a new group of cephalosporins having excellent broad spectrum activity.

In accordance with the invention a semisynthetic cephalosporin of Formula (1)

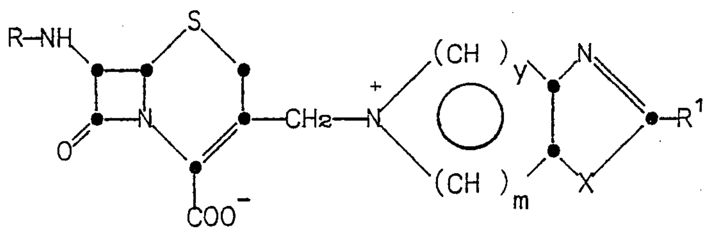 (1)

in which R is hydrogen, formyl, α-aminoadipoyl, protected α-aminoadipoyl, or an acyl group represented by the formula

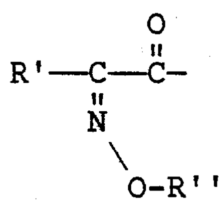

in which R' is a 5- or 6-membered heterocyclic ring represented by the formulae:

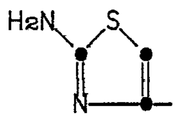 , 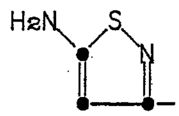 , 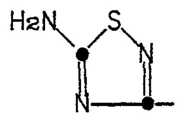 , 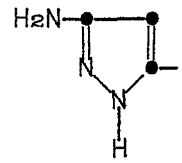 ,

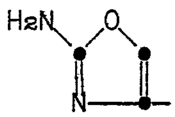 , 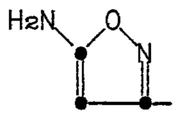 , 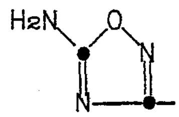 , 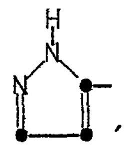 ,

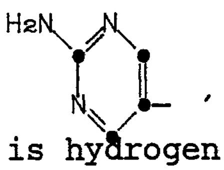 , 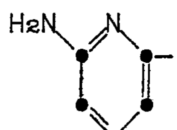 , or 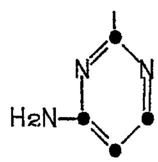 ;

R'' is hydrogen, $C_1$-$C_4$ alkyl, a carboxy-substituted alkyl or carboxy-substituted cycloalkyl group represented by the formula

$$-\underset{b}{\overset{a}{C}}-(CH_2)_n-COR'''$$

in which n is 0-3, a and b when taken separately are, independently, hydrogen or $C_1$-$C_3$ alkyl, or when taken together with the carbon to which they are attached form a $C_3$-$C_7$ carbocyclic ring; R''' is hydroxy, amino, $C_1$-$C_4$ alkoxy, or OR° in which R° is indanyl, phthalidyl, an acyloxymethyl group of the formula $-CH_2-OC(O)R_2$, in which $R_2$ is $C_1$-$C_4$ alkyl or phenyl; or -COOR° is a protected carboxy group;

or R'' is an N-substituted carbamoyl group represented

by the formula

$$-\overset{\displaystyle O}{\overset{\|}{C}}-NHR''''$$

in which R'''' is $C_1$-$C_4$ alkyl, phenyl, or $C_1$-$C_3$ alkyl substituted by phenyl;

y and m, independently, are integers equal to 0, 1, 2 or 3, provided that y plus m equals 3;

$R^1$ is hydrogen, $C_1$-$C_4$ alkyl, phenyl, thienyl, amino or $C_1$-$C_4$ alkanoylamino;

X is O, S or N-$R^2$, where $R^2$ is hydrogen or $C_1$-$C_4$ alkyl; or a pharmaceutically-acceptable salt or biologically-cleavable ester thereof, is useful as a broad spectrum antibiotic.

A class of such compounds of Formula (1) are those in which R is hydrogen, formyl, α-aminoadipoyl, protected α-aminoadipoyl, or an acyl group of the formula

$$R'-\underset{\underset{\displaystyle N\diagdown O-R''}{\|}}{C}-\overset{\displaystyle O}{\overset{\|}{C}}-$$

in which R' is a 5- or 6-membered heterocyclic ring of the formulae

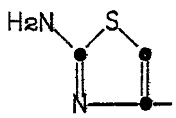 , 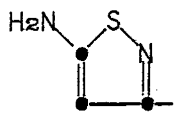 , 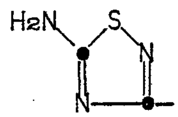 , 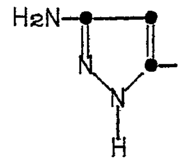 ,

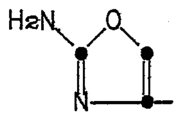 , 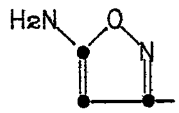 , 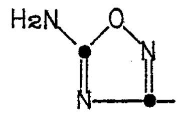 , 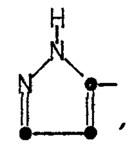 ,

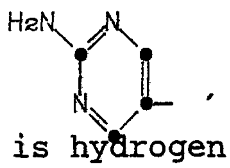 , 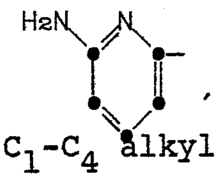 , or 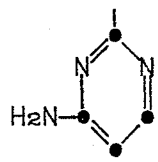 ;

R'' is hydrogen, $C_1$-$C_4$ alkyl, a carboxy-substituted alkyl or carboxy-substituted cycloalkyl group of the formula:

$$-\overset{a}{\underset{b}{C}}-(CH_2)_n-COR'''$$

in which n is 0-3; a and b when taken separately are, independently, hydrogen or $C_1$-$C_3$ alkyl, and when taken together with the carbon to which they are bonded form a $C_3$-$C_7$ carbocyclic ring; R''' is hydroxy, $C_1$-$C_4$ alkoxy, amino, or OR°, in which R° is indanyl, phthalidyl, or an acyloxymethyl group of the formula $-CH_2-O-C(O)-R_2$ $R_2$ is $C_1$-$C_4$ alkyl or phenyl; or COOR° is a protected carboxy group;

or R'' is an N-substituted carbamoyl group of the formula

$$-\overset{\overset{\displaystyle O}{\|}}{C}-NHR''''$$

in which R'''' is $C_1$-$C_4$ alkyl, phenyl or $C_1$-$C_3$ alkyl substituted by phenyl;

y and m, independently, are integers equal to 0, 1, 2 or 3, provided that y plus m equals 3;

$R^1$ is hydrogen, $C_1$-$C_4$ alkyl, phenyl, thienyl, amino or $C_1$-$C_4$ alkanoylamino;

X is O, S or N-$R^2$, where $R^2$ is hydrogen or $C_1$-$C_4$ alkyl; or a pharmaceutically-acceptable salt or biologically-cleavable ester thereof, provided that when R' is

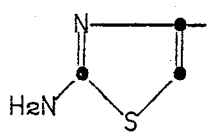 ;

R'' is $C_1$-$C_4$ alkyl, or n=0, a and b, independently, are hydrogen, methyl, ethyl or when a and b are taken together with the carbon to which they are attached form a $C_3$-$C_5$ carbocyclic ring; y=1 and m=2; and X=S, then $R^1$ may only be phenyl, thienyl or $C_1$-$C_4$ alkanoylamino.

In a preferred embodiment, R is an acyl group of the formula

$$R'-\underset{\underset{\displaystyle N\diagdown OC_1\text{-}C_4alkyl.}{\|}}{C}-\overset{\overset{\displaystyle O}{\|}}{C}-$$

Within this group, R' is preferably

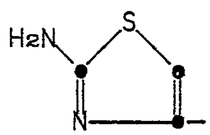

.

In another preferred embodiment, X in the above formula is N-$R^2$ or S. Also preferred are compounds in which y is 1 and m is 2.

Further, in accordance with the invention, there is provided a process for preparing a compound of Formula (1) as defined earlier, or a pharmaceutically-acceptable salt or biologically-cleavable ester thereof, which comprises:

(a) condensing a compound of Formula (2):

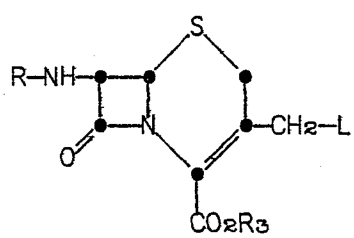

(2)

in which L is a leaving group, $R_3$ is hydrogen or a carboxy-protecting group, and R is as defined earlier with a bicyclicpyridine compound of the Formula:

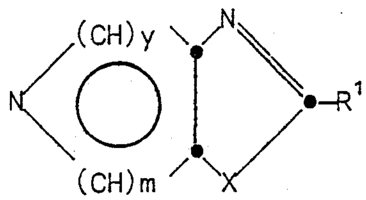

,

in which y, m, x and $R^1$ are defined above, removing any protecting group which may be present, and/or salifying or esterifying the product.

(b) acylating a compound of Formula (1) in which R is hydrogen, or a salt or 4'-ester thereof, with an acid of the Formula:

```
R'-C-COOH
   "
   N-OR''
```

or an activated derivative thereof, in which R' and R" are defined earlier and if desired, removing any protecting group present and/or salifying or esterifying the product.

(c) deacylating a compound of Formula (1) in which R is other than hydrogen, or a salt or ester thereof to form a compound in whcih R is hydrogen, or a salt or ester thereof.

This invention also provides pharmaceutical formulations comprising as an active ingredient a cephalosporin as defined above and a pharmaceutical carrier, excipient or diluent therefor.

Also provided is a method of treating bacterial infections in animals employing a compound provided by this invention.

When used, "$C_1$-$C_4$ alkyl" refers to methyl, ethyl, n-propyl, isopropyl, n-butyl, t-butyl, sec-butyl, and similar groups; "$C_1$-$C_4$ alkoxy" refers to groups such as methoxy, ethoxy, n-propoxy, isopropoxy, n-butoxy, t-butoxy, and sec-butoxy; "$C_1$-$C_3$ alkyl" refers to methyl, ethyl, n-propyl, and isopropyl; "$C_1$-$C_3$ alkyl substituted by phenyl" refers to groups such as benzyl, 2-phenethyl, 1-phenethyl, 3-phenylpropyl, and 2-phenylpropyl; and "$C_3$-$C_7$ carbocyclic ring" refers to groups such as cyclopropyl, cyclobutyl, cyclopentyl, cyclohexyl, and cycloheptyl; and "$C_1$-$C_4$ alkanoylamino" refers to groups such as formamido, acetamido, propionamido, and butyramido.

The term "protected α-aminoadipoyl" refers to the α-aminoadipoyl acyl group in which the amino group and the carboxy group are blocked or protected with conventional protecting groups. For example, the amino group can be protected with an acyl or haloacyl group such as acetyl, chloroacetyl, propionyl, benzoyl, chlorobenzoyl, dichloro or dibromobenzoyl, phthaloyl, 2-carboxytetrachlorobenzoyl, or 2-carboxytetrabromobenzoyl; or an alkyloxycarbonyl or aryloxycarbonyl group such as ethoxycarbonyl, trichloroethoxycarbonyl, t-butyloxycarbonyl, t-amyloxycarbonyl, benzyloxycarbonyl, or p-nitrobenzyloxycarbonyl. Conventional carboxy-protecting groups are, for example, the ester forming groups commonly employed in the β-lactam antibiotic art to block or protect the acidic carboxy group during the preparation of the antibiotic compounds. Examples of

such groups are described later for the definition of the term R° of Formula (1).

The carboxy-substituted alkyl and carboxy-substituted cycloalkyl groups represented by R'' in Formula (1) when R''' is hydroxy are exemplified by groups such as carboxymethyl, 2-carboxyethyl, 3-carboxypropyl, 4-carboxybutyl, 2-carboxyprop-2-yl, 2-carboxyprop-1-yl, 2-methyl-4-carboxybut-2-yl, 3-carboxy-3-methylprop-2-yl, 1-carboxycycloprop-1-yl, 1-carboxycyclobut-1-yl, 1-carboxycyclopent-1-yl, 1-carboxycyclohex-1-yl, 1-carboxymethylcyclobut-1-yl, or 2-carboxyethylcyclohex-1-yl. When in the above formula R''' is $NH_2$, examples of the carboxamides represented are the amides of the above-named carboxy-substituted radicals.

The esters of the carboxy-substituted groups (Formula (1), R'' is carboxy-substituted alkyl or cycloalkyl and R''' is $C_1$-$C_4$ alkoxy) are illustrated by methoxycarbonylmethyl, ethoxycarbonylmethyl, 2-(ethoxycarbonyl)prop-2-yl, 1-propoxycarbonylcyclopent-1-yl, and similar $C_1$-$C_4$ alkyl esters of the above-named carboxy-substituted alkyl and cycloalkyl radicals.

Examples of N-substituted carbamoyl groups (e.g. Formula (1), R'' is carbamoyl) are N-methylcarbamoyl, N-ethylcarbamoyl, N-propylcarbamoyl, N-phenylcarbamoyl, or N-benzylcarbamoyl.

The compounds of this invention are characterized in part by the bicyclic pyridinium group attached to the 3-methyl group of the cephalosporin nucleus. Typical bicyclic pyridines which may be employed in the synthesis of the pyridinium-methyl derivatives of this

invention are illustrated below. The numbering system employed in the naming of the compounds of the invention is indicated in the following formulae:

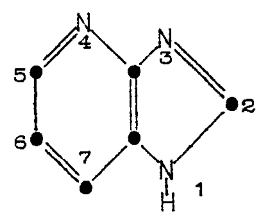

1H-imidazolo[4,5-b]-
pyridine

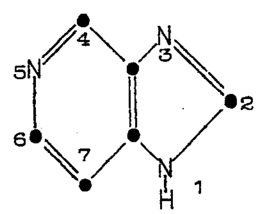

3H-imidazolo[4,5-c]-
pyridine

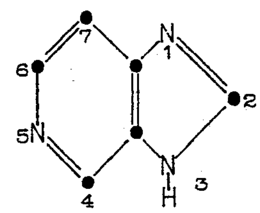

3H-imidazolo[4,5-c]-
pyridine

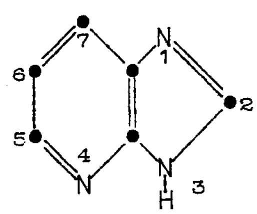

3H-imidazolo[4,5-b]pyridine

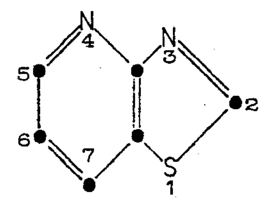

thiazolo[4,5-b]pyridine

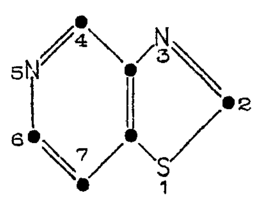

thiazolo[4,5-c]pyridine

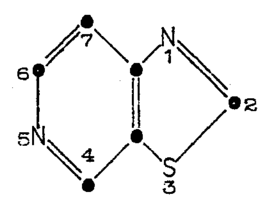

thiazolo[5,4-c]pyridine

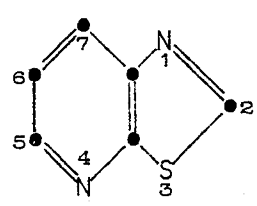

thiazolo[5,4-b]pyridine

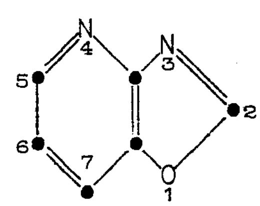

oxazolo[4,5-b]pyridine

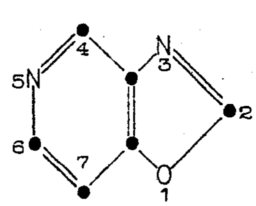

oxazolo[4,5-c]-
pyridine

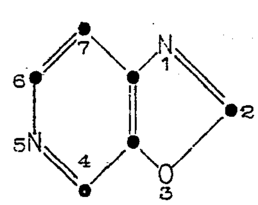

oxazolo[5,4-c]-
pyridine

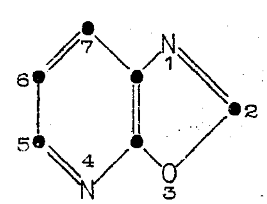

oxazolo[5,4-b]-
pyridine

The imidazolopyridines, oxazolopyridines and thiazolopyridines, the required starting materials, are known compounds which are synthesized employing procedures known in the art.

Carboxy-protected derivatives of the compounds represented by the above formula when R'' is a carboxy-substituted alkyl or carboxy-substituted cycloalkyl group and R''' is OR°, are esters of the carboxy group commonly known in the art as carboxy-protecting or blocking groups. Examples of such ester groups (-COOR°) are those in which R° is alkyl, alkenyl, and substituted alkyl ester groups such as t-butyl, 2-methylbutene-2-yl, 2,2,2-trichloroethyl, 2,2,2-tribromoethyl, and 2-iodoethyl; the benzyl ester and substituted benzyl esters such as p-methoxybenzyl and p-nitrobenzyl; the diphenylmethyl ester and substituted diphenylmethyl esters such as the 4-methoxydiphenylmethyl and 4,4'-dimethoxydiphenylmethyl esters; and trialkylsilyl esters such as trimethylsilyl, and other similar ester groups. The carboxy-protecting group is used for the temporary protection of the carboxy group, for example, during the preparation of the compounds of Formula (1). These groups are removed readily under hydrolytic or hydrogenolytic conditions generally known in the art.

The esters defined by Formula (1), when R'' is a carboxy-substituted alkyl or a carboxy-substituted cycloalkyl group and R''' is OR°, namely the indanyl, phthalidyl, and acyloxymethyl esters, are biologically-cleavable esters. Examples of such esters are the 5-indanyl, phthalidyl, acetoxymethyl, propionoxymethyl,

pivaloyloxymethyl, and benzoyloxymethyl esters. The biologically-cleavable esters are obtained by reacting the carboxylic acid function in the salt form, e.g. the sodium or potassium salt, with bromophthalide, or with an acyloxymethyl halide, e.g. acetoxymethyl bromide or pivaloyloxymethyl bromide. The indanyl ester is prepared with 5-indanol, the cephalosporin acid and a condensing agent such as DCC or EEDQ.

The heterocyclic rings represented by R' in Formula (1) are named as follows: 2-aminothiazol-4-yl, 5-aminoisothiazol-3-yl, 5-amino-1,2,4-thiadiazol-3-yl, pyrazol-5-yl, 3-aminopyrazol-5-yl, 2-aminopyrimidin-5-yl, 4-aminopyrimidin-2-yl, 2-aminopyridin-6-yl, 2-aminooxazol-4-yl, 5-aminoisoxazol-3-yl, and 5-amino-1,2,4-oxadiazol-3-yl.

To describe the compounds of the invention, the term "oximino" refers to the oxime and substituted oxime function

$$\begin{array}{l} -C- \\ \;\;\| \\ \;\;N-O-R'' \end{array} .$$

The compounds of the invention in which R is an acyl group of the formula

$$\begin{array}{l} \qquad\quad O \\ \qquad\quad \| \\ R'-C-C- \\ \qquad \| \\ \qquad N \\ \qquad\;\; \backslash O-R'' \end{array}$$

are broad spectrum antibiotics which inhibit the growth of microorganisms pathogenic to man and animals. For example, these compounds are effective in controlling

the growth of staphylococci, streptococci and penicillin-resistant strains of staphylococci. They also inhibit the growth of gram-negative bacteria, for example Proteus, Pseudomonas, Enterobacter, Escherichia coli, Klebsiella, Shigella, Serratia, and Salmonella.

As described later, the compounds represented by Formula (I) in which R is hydrogen, formyl, aminoadipoyl, or protected aminoadipoyl are intermediates useful in the preparation of the compounds in which R is an acyl group.

The compounds in which R is an acyl group are prepared by the reaction of a bicyclicpyridine(imidazolo-pyridine, oxazolopyridine or thiazolopyridine) with a 7-acylaminocephalosporin represented by Formula (2a)

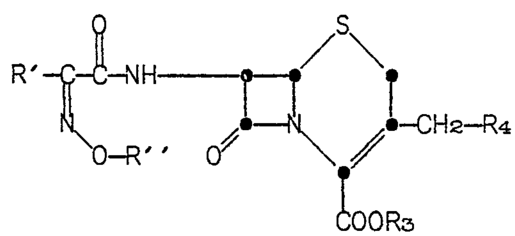

(2a)

in which R' and R'' have the same meanings as defined earlier, $R_3$ is hydrogen or a carboxy-protecting group, and $R_4$ is a leaving group, preferably chloro, bromo, iodo, or acetoxy. The displacement reaction preferably is carried out with a compound of Formula (2a) in which $R_4$ is acetoxy or iodo. In a preferred method, a compound in which $R_4$ is iodo is prepared first by reacting, by the method of Bonjouklian, U.S. Patent No. 4,266,049 issued May 5, 1981, a compound in which $R_4$ is acetoxy

and $R_3$ is an ester group, with trimethylsilyliodide (trimethyliodosilane, TMSI). The 3-iodomethyl cephalosporin then is reacted with the bicyclicpyridine to provide a compound of the invention.

T perform the process, a compound of Formula (2a) in which $R_4$ is acetoxy first is silylated to form the silyl ester of the C-4 carboxy group and silyl derivatives of other silyl reactive groups. The silylation is carried out at room temperature in an aprotic organic solvent with a silylating reagent such as mono- or bis- trimethylsilylacetamide, mono-trimethylsilyltrifluoroacetamide, or N-methyl-N-trimethylsilyltrifluoroacetamide. The silylated derivative then is reacted at ambient temperature with trimethylsilyliodide to provide the silylated 3-iodomethyl cephalosporin. The silylated 3-iodomethyl cephalosporin then is reacted with the bicyclicpyridine to provide a silylated compound of the invention. Hydrolysis of the silyl groups provides the final desired compound of Formula (I).

The process is illustrated by the following reaction scheme in which a trimethyl silylating reagent and a 1H-imidazolo[4,5-c]pyridine are used.

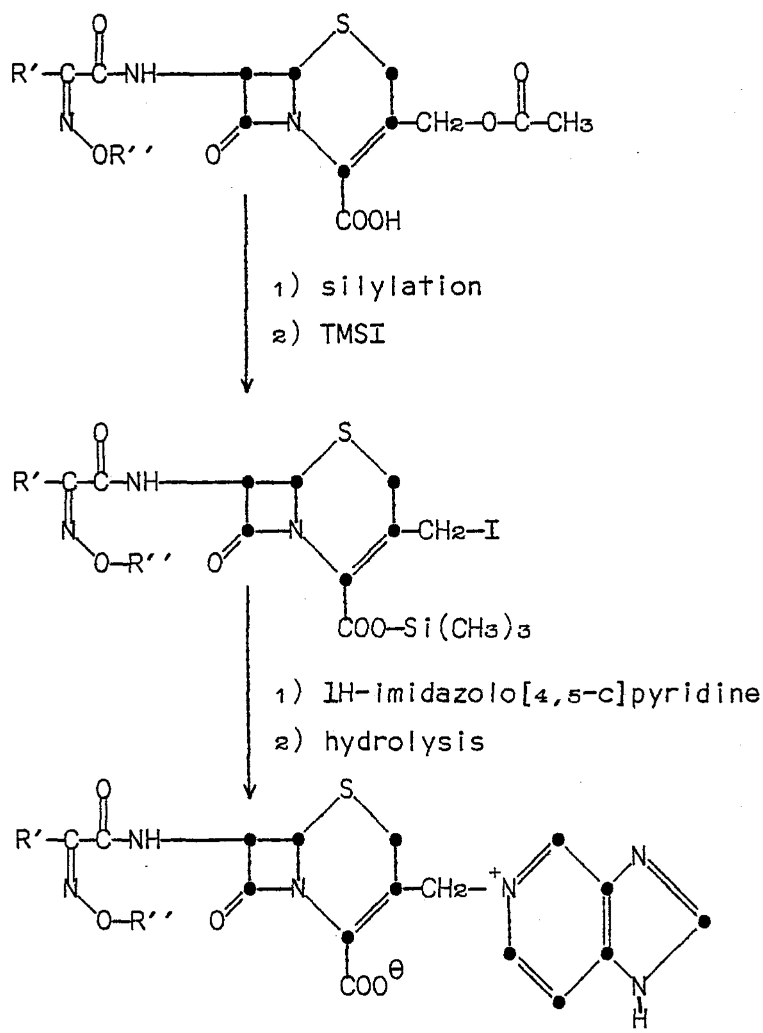


In this scheme, R' and R'' have the same meanings as defined earlier.

Alternatively, the antibiotic compounds of the invention are prepared directly from a 3-acetoxymethyl

cephalosporin compound (e.g. $R_4$ is acetoxy, $R_3$ is H) by displacement of the acetoxy group with the bicyclicpyridine. The preparation is performed in a known manner, for instance, in an aqueous medium, for example in a water miscible organic solvent containing water. The addition of a small amount of an alkali metal iodide, such as potassium iodide, can enhance the rate of the reaction. The reaction is carried out at a temperature between about 35°C. and about 70°C. Water miscible organic solvents such as acetone, acetonitrile, tetrahydrofuran, and dimethylacetamide are useful solvents.

This invention also provides compounds of Formula (1) as salts formed with strong acids and the salt form of biologically-labile esters. Such salts are represented by Formula (3):

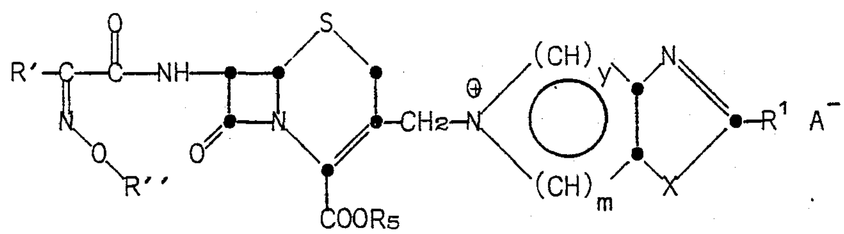 (3)

in which R', R'', and $R^1$ are as defined earlier and $R_5$ is hydrogen, indanyl, phthalidyl, or an acyloxymethyl group of the formula

$$-CH_2-O-\overset{O}{\overset{\|}{C}}-R_2$$

in which $R_2$ is as defined earlier; and $A^-$ is an anion such as chloride, bromide, iodide, sulfate, or phosphate.

Examples of acyloxymethyl ester groups, $R_5$, are acetoxymethyl, propionoxymethyl, pivaloyloxymethyl, and benzoyloxymethyl groups.

A compound of Formula (1) is converted to its strong acid salt by reaction with about one molar equivalent or excess of an acid such as hydrochloric acid, hydrobromic acid, sulfuric acid, or phosphoric acid.

The biologically-labile esters are prepared with a compound of Formula (1) and an acyloxymethyl halide, an indanyl halide, for example, 5-bromoindane or phthalidyl bromide. Upon esterification, the salt form of the ester is obtained. For example, with acetoxymethyl bromide the acetoxymethyl ester bromide is obtained (Formula (3), $R_5$ is acetoxymethyl, $A^-$ is $Br^-$).

One skilled in the art will appreciate that if in a compound of Formula (1) R'' is a carboxy-substituted alkyl or cycloalkyl group and R''' is hydroxy, the di-biologically-labile esters may be prepared. Likewise, acid addition salts will be formed with any basic amino groups present in the molecule (i.e. Formula (1) in which an amino-substituted heterocyclic group is present) when the strong acid salts of Formula (3) are prepared.

The biologically-labile ester salts and the strong acid salts represented by Formula (3) are alternative forms of the compounds of Formula (1) and may be formulated for administration in therapy.

The compounds of Formula (1), in which R is hydrogen or formyl, are prepared with 7-aminocephalosporanic acid and 7-formamidocephalosporanic acid, respectively, by displacement of the 3-acetoxy group with the bicyclicpyridine as described above. Alternatively, 7-formamido-3-iodomethyl-3-cephem-4-carboxylic acid trimethylsilyl ester is prepared by the Bonjouklian method as described earlier and then is reacted with the bicyclicpyridine to provide the compound of Formula (1) in which R is formyl.

Alternatively, the 7-amino nucleus compounds of Formula (1) (R is H) are prepared by the well-known N-deacylation reaction which proceeds through an imino chloride to an imino ether and upon decomposition of the latter, to the 7-amino-3-bicyclicpyridinium-4-carboxylate. Initially, a 7-acylaminocephalosporanic acid, in which the 7-acyl group can be, for example, phenylacetyl, phenoxyacetyl or a heterocyclic acyl group such as thienylacetyl, is reacted with the bicyclicpyridine to form the 7-acylamino-3-bicyclicpyridinium-methyl)-3-cephem-4-carboxylate. Alternatively, the latter compound is obtained via the 7-acylamino-3-iodomethyl ester (Bonjouklian method) which is allowed to react with the bicyclicpyridine. The 7-acyl bicyclopyridinium compound then is treated with an imino halide-forming reagent such as phosphorus pentachloride in an inert solvent in the presence of an acid-binding agent such as a tertiary amine, e.g., diethylaniline, to provide the imino halide derivative of the 7-position acylamido group. Without isolation, the imino halide is treated with an alcohol,

alkanediol or benzyl alcohol to form the corresponding imino ether. Decomposition of the imino ether, for example by aqueous hydrolysis, provides the 7-amino nucleus compound.

In an example of the preparation of a 7-amino nucleus compound by this method, 7-(2-thienylacetamido)-cephalosporanic acid is reacted with 1H-imidazolo[4,5-b]pyridine to prepare 7-(2-thienylacetamido)-3-(1H-imidazolo[4,5-b]pyridinium-4-ylmethyl)-3-cephem-4-carboxylate. The latter is converted to the trimethylsilyl ester with trimethylchlorosilane in a halogenated hydrocarbon solvent in the presence of a weak base such as dimethylacetamide in an amount corresponding to a 4-5 molar excess. Solvents such as methylene chloride, trichloroethane, and chloroform are suitable. The solution of the silyl ester is cooled to a temperature of about -30°C. to about 0°C. and an imino halide-forming reagent such as phosphorus pentachloride is added. After imino halide formation is complete, a $C_1$-$C_4$ alkanol, an alkanediol, or a benzyl alcohol is added to the cold reaction mixture. The reaction mixture is allowed to warm to about room temperature and the product, 7-amino-3-(1H-imidazolo[4,5-b]pyridinium-4-ylmethyl)-3-cephem-4-carboxylic acid, precipitates in the form of the dihydrochloride salt represented by the formula:

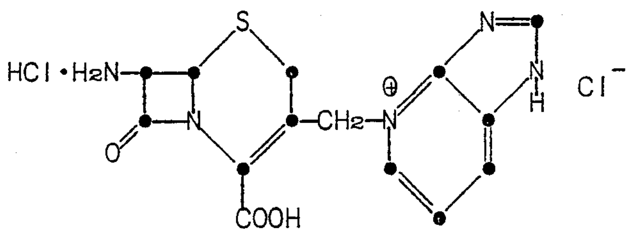

The N-formyl compounds (Formula (1), R is formyl) are useful as intermediates in the preparation of the antibiotic compounds of the invention. For example, 7-formamidocephalosporanic acid is silylated and the silyl ester converted to the 3-iodomethyl derivative with trimethylsilyliodide as described hereinabove. The 3-iodomethyl silylated derivative is reacted with the bicyclicpyridine to form the compound represented by Formula (1). The N-formyl-3-bicyclicpyridinium-methyl-3-cephem is then converted to the 7-amino nucleus compound with methanolic hydrochloric acid.

The 7-amino-3-(bicyclicpyridinium methyl)-3-cephem-4-carboxylate or the dihydrochloride salt thereof also is obtained with cephalosporin C in which the amino and carboxy groups are protected. For example, cephalosporin C is first silylated with a conventional silylating reagent such as N-methyl-N-trimethylsilyltrifluoroacetamide to form the N-trimethylsilyl di-trimethylsilyl ester derivative. The latter is reacted with TMSI by the Bonjouklian methód, and the 3-iodo-methyl silylated derivative of cephalosporin C obtained

then is allowed to react with the bicyclicpyridine, and, following hydrolysis of the silyl groups, the compound of the Formula (1) in which R is α-aminoadipoyl is obtained. The α-aminoadipoyl side chain is cleaved by the N-deacylation procedure described above. In carrying out the N-deacylation, the amino group and the carboxy groups of the molecule are protected.

In carrying out the preparation of a 7-amino-3-(bicyclicpyridinium methyl)-3-cephem-4-carboxylate with Cephalosporin C, the silylated 3-(bicyclicpyridinium methyl) derivative obtained in the Bonjouklian method as described above can be used. Because the amino group and the two carboxy groups are silylated, and thus protected, the N-deacylation can be performed directly. During the final step of the N-deacylation, i.e. following the formation of the imino ether of the side chain moiety, water is added to effect the hydrolysis of the silyl protecting group. This preparation is illustrated by the following reaction scheme.

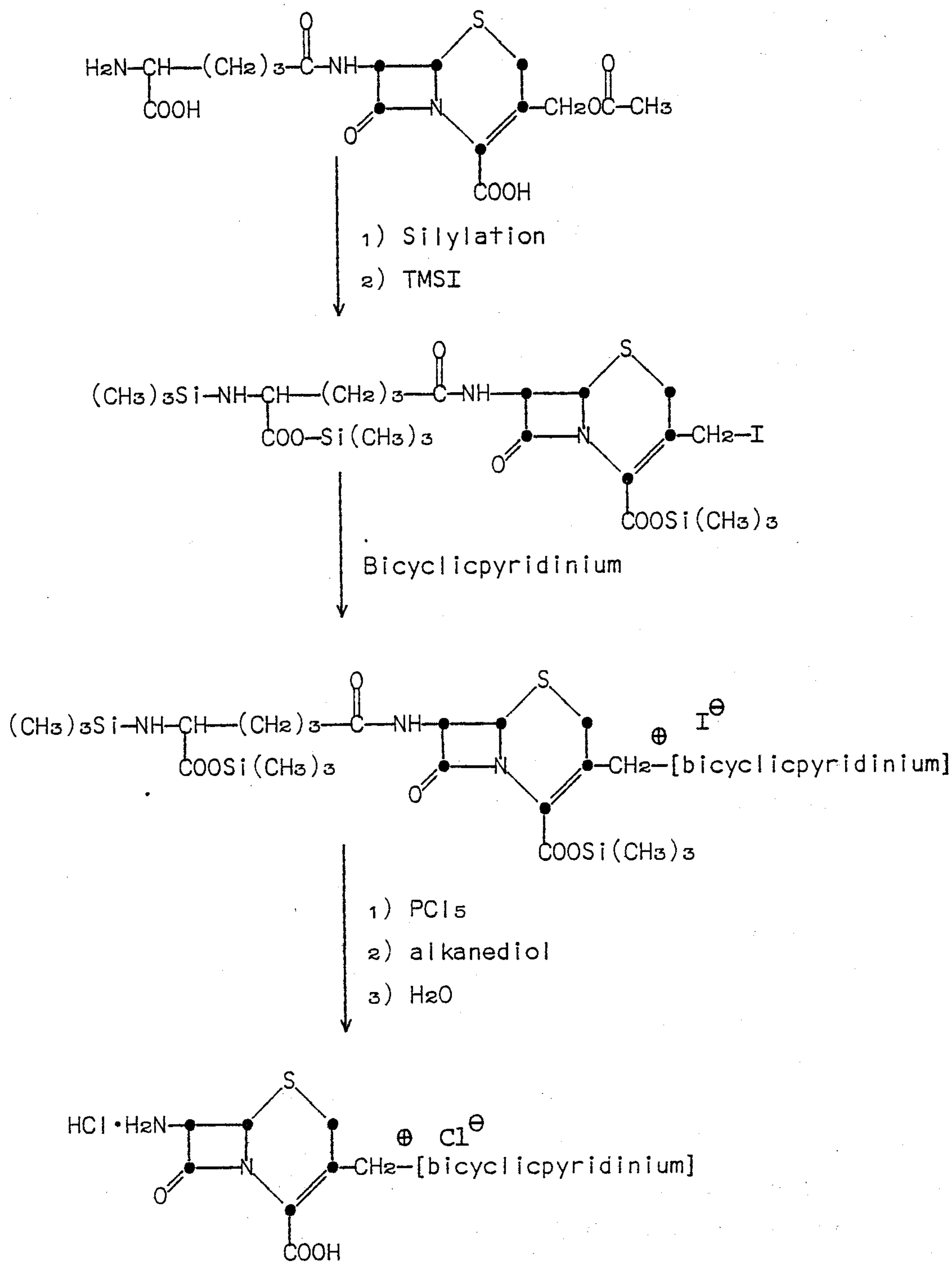
1) Silylation
2) TMSI
Bicyclicpyridinium
1) PCl5
2) alkanediol
3) H2O

Alternatively, the 7-amino-3-(bicyclicpyridinium methyl) nucleus compound can be obtained with Cephalosporin C having the amino group and the carboxy groups protected. Examples of protecting groups which can be used are given earlier for the definition of the term "protected aminoadipoyl".

The 7-amino nucleus compound (Formula (1), R = H) prepared by the N-deacylation method or via the N-formyl derivative is acylated with a 2-(heterocyclic)-2-oximinoacetic acid represented by the Formula

```
R'-C-COOH
   ‖
   N
    \O-R''
```

or an activated derivative thereof, to provide an antibiotic compound of Formula (1). The N-acylation coupling reaction is performed using acylation methods well-known in the art. Active derivatives of the carboxy group such as the so-called "active esters" can be used. Examples of active esters are those formed with the oximino acetic acid and hydroxybenzotriazole (HBT), or hydroxysuccinimide; and the esters formed with methyl chloroformate and isobutyl chloroformate. The acylation also can be carried out by employing the acid halide, <u>e.g.</u> the acid chloride, in the presence of an acid scavenger such as sodium bicarbonate or triethylamine.

The amino group of the amino-substituted heterocycles (R' in formula 1) desirably is protected during the N-acylation of the 7-amino nucleus compound. Amino-protecting groups which can be used are those

commonly employed in the cephalosporin art for the temporary protection of basic amino groups to block or prevent the amino group from interfering with a reaction carried out at another site in the molecule. Examples of such groups are the haloacyl groups such as chloroacetyl and dichloroacetyl; the urethane-forming protecting groups such as t-butyloxycarbonyl, 2,2,2-trichloroethoxycarbonyl, cyclopentyloxycarbonyl, adamantyloxycarbonyl, benzyloxycarbonyl, p-nitrobenzyloxycarbonyl, and diphenylmethyloxycarbonyl; and other protecting groups such as trityl (triphenylmethyl) and benzhydryl.

The compounds represented by Formula (2a) in which $R_4$ is an acetoxy group are prepared by known methods. For example, compounds in which R' is the 2-aminothiazol-4-yl group are described by Heymes et. al., U.S. Patent No. 4,152,432; compounds in which R' is 2-aminopyridin-6-yl, 2-aminopyrimidin-5-yl, or 4-aminopyrimidin-2-yl, are described in U.S. Patent No. 4,167,176; compounds in which R' is 5-amino-1,2,4-thiadiazol-3-yl are described in EPO Publication No. 0,007,470; compounds in which R' is 2-aminooxazol-4-yl, 5-amino-1,2,4-oxadiazol-3-yl or 5-aminoisoxazol-3-yl are described in U.S. Patent No. 4,406,898; compounds in which R'' is an N-substituted carbamoyl group are prepared by methods described in U.S. Patent No. 4,200,575; and compounds in which R' is 3-aminopyrazol-5-yl, or pyrazol-5-yl are obtained as described in U.K. Patent Application 2,046,734A.

Commonly, the compounds of Formula (2a) in which $R_4$ is acetoxy are prepared by the N-acylation of

the 7-amino group of 7-aminocephalosporanic acid, or an ester thereof, with the 2-(heterocyclic)-2-oximinoacetic acid by employing acylation methods known in the art. For example, the heterocyclic oximino-substituted acetic acid is converted to an active ester, such as the ester formed with hydroxybenzotriazole or hydroxysuccinimide, and the active ester is used as the acylating moiety. Other active derivatives of the carboxylic acid such as the acid chloride or acid azide can be used in the acylation.

The compounds of Formula (2) in which R' is a pyrazol-5-yl or 3-aminopyrazol-5-yl group are prepared by employing methods known in the art. The 2-(pyrazol-5-yl)-2-oximinoacetic acid or the 2-(3-aminopyrazol-5-yl)-2-oximinoacetic acid is prepared and converted to an active derivative of the carboxylic acid, for example, an active ester. The active ester is used to N-acylate 7-aminocephalosporanic acid. The resulting 7-[2-(pyrazol-5-yl)-2-oximinoacetamido]-3-acetoxymethyl-3-cephem-4-carboxylic acid and 7-[2-(3-aminopyrazol-5-yl)-2-oximinoacetamido]-3-acetoxymethyl-3-cephem-4-carboxylic acid are converted to the corresponding 3-iodomethyl silylated derivatives as described earlier. The latter may be reacted with, for example, thienopyridine to provide the respective compound of the invention.

The pyrazole and aminopyrazole oximino substituted acetic acids are prepared by synthetic methods known in the art. For example, the 2-(pyrazol-5-yl)-2-alkoxyiminoacetic acid is prepared by heating in an

inert hydrocarbon solvent the acetyl oximino compound of formula (A):

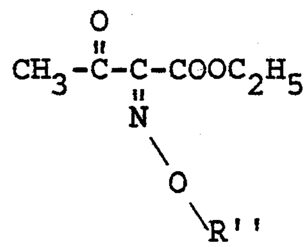

(A)

,

in which R'' is as defined above but is other than hydrogen, with dimethylformamide dimethylacetal to form the dimethylaminomethylene oximino ester of the formula

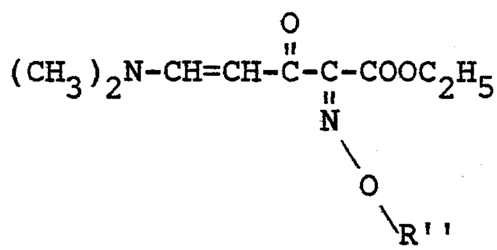

The latter is reacted with hydrazine hydrate to provide the ethyl ester of 2-(pyrazol-5-yl)-2-alkoxyiminoacetic acid. The ester is hydrolyzed to the free acid and the acid converted to an active ester for acylation.

The 2-(3-aminopyrazol-5-yl)-2-alkoxyimino-acetic acid is prepared by reacting the compound of formula (A) with carbon disulfide and two equivalents of methyl iodide to form the intermediate compound of formula (B)

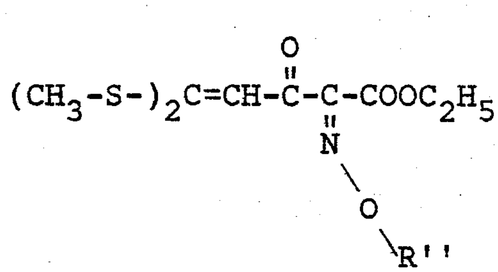

(B)

Intermediate (B) is reacted with N-t-BOC hydrazine to provide compound (C),

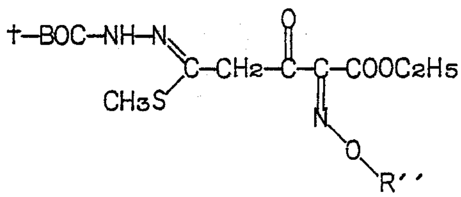

(C)

and (C) is reacted with hydrazine hydrate to form 2-(3-t-BOC-hydrazinopyrazol-5-yl)-2-oximinoacetic acid ethyl ester (D).

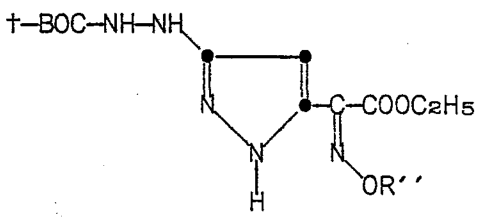

(D)

Compound (D) is treated with trifluoroacetic acid to remove the t-BOC group and the 3-hydrazinopyrazole is nitrosated with nitrous ($HNO_2$) acid, in the cold, to form 2-(3-azidopyrazol-5-yl)-2-oximinoacetic acid ethyl ester. The azido group is reduced to the amino group by chemical reduction to provide the 2-(3-aminopyrazol-5-yl)-oximinoacetic acid ethyl ester. The ester is hydrolyzed under alkaline conditions to the free acid.

The compounds of the invention have the same stereochemistry as known cephalosporin antibiotics. The 7-position side chain has the natural β-configuration (6R, 7R), while the oximino group in the side chain can exist in the <u>syn</u> or <u>anti</u> forms or as a mixture of both.

Compounds of the invention in either form are prepared by employing the 2-(heterocyclic)-2-oximinoacetic acid acylating moiety in the syn or anti form. Alternatively, mixtures of the syn and anti compounds of Formula (1) can be separated by chromatographic means such as by HPLC. The compounds in the syn form are preferred because of their higher activity.

Examples of bicyclicpyridinium compounds of Formula (1) in which R is an acyl group may include the following compounds:

7-[2-(2-aminothiazol-4-yl)-2-hydroxyimino-acetamido]-3-(1H-imidazolo[4,5-b]pyridinium-4-ylmethyl)-3-cephem-4-carboxylate,

7-[2-(2-aminothiazol-4-yl)-2-methoxyimino-acetamido]-3-(1H-imidazolo[4,5-c]pyridinium-5-ylmethyl)-3-cephem-4-carboxylate,

7-[2-(2-aminothiazol-4-yl)-2-ethoxycarbonyl-methoxyiminoacetamido]-3-(3H-imidazolo[4,5-c]pyridinium-5-ylmethyl)-3-cephem-4-carboxylate,

7-[2-(2-aminothiazol-4-yl)-2-methoxyimino-acetamido]-3-(3H-imidazolo[4,5-b]pyridinium-4-ylmethyl)-3-cephem-4-carboxylate,

7-[2-(2-aminothiazol-4-yl)-2-methoxyimino-acetamido]-3-(thiazolo[4,5-c]pyridinium-5-ylmethyl)-3-cephem-4-carboxylate,

7-[2-(2-aminothiazol-4-yl)-2-methoxyimino-acetamido]-3-(thiazolo[4,5-b]pyridinium-4-ylmethyl)-3-cephem-4-carboxylate,

7-[2-(2-aminothiazol-4-yl)-2-methoxyiminoacetamido]-3-(thiazolo[5,4-c]pyridinium-5-ylmethyl)-3-cephem-4-carboxylate,

7-[2-(2-aminothiazol-4-yl)-2-methoxyiminoacetamido]-3-(thiazolo[5,4-b]pyridinium-4-ylmethyl)-3-cephem-4-carboxylate,

7-[2-(2-aminopyridin-6-yl)-2-(2-carboxypropyl)xyiminoacetamido)-3-(oxazolo[4,5-b]pyridinium-4-ylmethyl)-3-cephem-4-carboxylate,

7-[2-(5-amino-1,2,4-thiadiazol-3-yl)-2-methoxyiminoacetamido]-3-(oxazolo[4,5-c]pyridinium-5-ylmethyl)-3-cephem-4-carboxylate,

7-[2-(5-aminoisothiazol-3-yl)-2-(2-carboxyprop-2-yl)oxyiminoacetamido]-3-(oxazolo[5,4-c]pyridinium-5-ylmethyl)-3-cephem-4-carboxylate,

7-[2-(2-aminopyrimidin-5-yl)-2-ethoxyiminoacetamido]-3-(oxazolo[5,4-b]pyridinium-4-ylmethyl)-3-cephem-4-carboxylate,

7-[2-(4-aminopyrimidin-2-yl)-2-(N-methylcarbamoyloxy)iminoacetamido]-3-(1H-2-methylimidazolo[4,5-b]pyridinium-4-ylmethyl)-3-cephem-4-carboxylate,

7-[2-(2-aminopyridin-6-yl)-2-methoxyiminoacetamido]-3-(3H-2-phenylimidazolo[4,5-b]pyridinium-4-ylmethyl)-3-cephem-4-carboxylate,

7-[2-(3-aminopyrazol-5-yl)-2-methoxyiminoacetamido]-3-(2-ethylthiazolo[5,4-c]pyridinium-5-ylmethyl)-3-cephem-4-carboxylate,

7-[2-(2-aminooxazol-4-yl)-2-ethoxycarbonylmethoxyiminoacetamido]-3-(2-aminooxazolo[5,4-c]pyridinium-5-ylmethyl)-3-cephem-4-carboxylate,

7-[2-(pyrazol-5-yl)-2-methoxycarbonylmethoxyiminoacetamido]-3-(2-acetamidothiazolo[5,4-c]pyridinium-5-ylmethyl)-3-cephem-4-carboxylate,

7-[2-(pyrazol-5-yl)-2-ethoxycarbonylmethoxyiminoacetamido]-3-(2-(2-thienyl)oxazolo[5,4-c]pyridinium-5-ylmethyl)-3-cephem-4-carboxylate,

7-[2-(5-aminoisoxazol-3-yl)-2-methoxyiminocetamido]-3-(2-aminooxazolo[5,4-b]pyridinium-4-ylmethyl)-3-cephem-4-carboxylate,

7-[2-(2-aminothiazol-4-yl]-2-(2-carboxyprop-2-yl)oxyiminoacetamido]-3-(1H-2-aminoimidazolo[4,5-c]pyridinium-5-ylmethyl)-3-cephem-4-carboxylate,

7-[2-(2-aminothiazol-4-yl)-2-(2-carboxyprop-2-yl)oxyiminoacetamido]-3-(1,2-dimethylimidazolo[4,5-c]pyridinium-5-ylmethyl)-3-cephem-4-carboxylate,

7-[2-(5-amino-1,2,4-oxadiazol-3-yl)-2-(2-carboxyprop-2-yl)oxyiminoacetamido]-3-(1-methyl-2-phenyl-3H-imidazolo[4,5-c]pyridinium-5-ylmethyl)-3-cephem-4-carboxylate,

7-[2-(2-aminothiazol-4-yl)-2-(2-carboxyprop-2-yl)oxyiminoacetamido]-3-(2-formamidothiazolo[4,5-b]pyridinium-4-ylmethyl)-3-cephem-4-carboxylate,

7-[2-(2-aminopyridin-6-yl)-2-methoxyiminoacetamido]-3-(2-aminothiazolo[4,5-c]pyridinium-5-ylmethyl)-3-cephem-4-carboxylate,

7-[2-(2-aminopyridin-6-yl)-2-methoxyiminoacetamido]-3-(2-n-butylthiazolo[5,4-c]pyridinium-5-ylmethyl)-3-cephem-4-carboxylate,

7-[2-(2-aminopyridin-6-yl)-2-ethoxycarbonylmethoxyiminoacetamido]-3-(2-aminothiazolo[5,4-b]pyridinium-4-ylmethyl)-3-cephem-4-carboxylate,

7-[2-(5-amino-1,2,4-thiadiazol-3-yl)-2-oximinoacetamido]-3-(2-phenyloxazolo[4,5-b]pyridinium-4-ylmethyl)-3-cephem-4-carboxylate,

7-[2-(2-aminothiazol-4-yl)-2-methoxyiminoacetamido]-3-(2-phenyloxazolo[5,4-c]pyridinium-5-ylmethyl)-3-cephem-4-carboxylate,

7-[2-(2-aminothiazol-4-yl)-2-methoxyiminoacetamido]-3-(2-isopropylthiazolo[5,4-b]pyridinium-4-ylmethyl)-3-cephem-4-carboxylate,

7-[2-(2-aminothiazol-4-yl)-2-methoxyiminoacetamido]-3-(2-ethyl-1-propyl-1H-imidazolo[4,5-b]-pyridinium-4-ylmethyl)-3-cephem-4-carboxylate,

7-[2-(2-aminothiazol-4-yl)-2-ethoxyiminoacetamido]-3-(1,2-dimethyl-3H-imidazolo[4,5-c]pyridinium-5-ylmethyl)-3-cephem-4-carboxylate,

7-[2-(pyrazol-5-yl)-2-ethoxycarbonylmethoxyiminoacetamido]-3-(2-(2-thienyl)thiazolo[4,5-b]pyridinium-4-ylmethyl)-3-cephem-4-carboxylate,

7-[2-(2-aminothiazol-4-yl)-2-methoxyiminoacetamido]-3-(2-phenyloxazolo[4,5-b]pyridinium-4-ylmethyl)-3-cephem-4-carboxylate,

7-[2-(5-aminoisoxazol-3-yl)-2-methoxyiminoacetamido]-3-(2-isopropyloxazolo[5,4-c]pyridinium-5-ylmethyl)-3-cephem-4-carboxylate,

7-[2-(2-aminothiazol-4-yl)-2-methoxyiminoacetamido]-3-(2-phenyloxazolo[5,4-b]pyridinium-4-ylmethyl)-3-cephem-4-carboxylate,

7-[2-(2-aminothiazol-4-yl)-2-methoxyimino-acetamido]-3-(2-aminooxazolo[4,5-c]pyridinium-5-yl-methyl)-3-cephem-4-carboxylate,

7-[2-(2-aminothiazol-4-yl)-2-methoxyimino-acetamido]-3-(2-aminothiazolo[4,5-c]pyridinium-5-yl-methyl)-3-cephem-4-carboxylate,

7-[2-(2-aminothiazol-4-yl)-2-(2-carboxyprop-2-yl)oxyiminoacetamido]-3-(2-isobutylthiazolo[5,4-c]pyridinium-5-ylmethyl)-3-cephem-4-carboxylate,

7-[2-(5-amino-1,2,4-thiadiazol-3-yl)-2-methoxyiminoacetamido]-3-(2-butyramido-1H-imidazolo[4,5-c]pyridinium-5-ylmethyl)-3-cephem-4-carboxylate,

7-[2-(pyrazol-5-yl)-2-ethoxycarbonylmethoxy-iminoacetamido]-3-[1,2-diethyl-3H-imidazolo[4,5-c]pyridinium-5-ylmethyl)-3-cephem-4-carboxylate,

7-[2-(2-aminothiazol-4-yl)-2-methoxyimino-acetamido]-3-(2-acetamidothiazolo[5,4-b]pyridinium-4-ylmethyl)-3-cephem-4-carboxylate,

7-[2-(2-aminothiazol-4-yl)-2-(2-carboxyprop-2-yl)oxyiminoacetamido]-3-(2-isobutyloxazolo[4,5-b]pyridinium-4-ylmethyl)-3-cephem-4-carboxylate,

7-[2-(2-aminopyrimidin-5-yl)-2-ethoxyimino-acetamido]-3-(2-methyloxazolo[5,4-b]pyridinium-4-yl-methyl)-3-cephem-4-carboxylate,

7-[2-(2-aminothiazol-4-yl)-2-hydroxyimino-acetamido]-3-(thiazolo[5,4-c]pyridinium-5-ylmethyl)-3-cephem-4-carboxylate,

7-[2-(3-aminopyrazol-5-yl)-2-methoxyimino-acetamido]-3-(2-phenylthiazolo[5,4-b]pyridinium-4-yl-methyl)-3-cephem-4-carboxylate,

7-[2-(2-aminothiazol-4-yl)-2-(2-carboxyprop-2-yl)oxyiminoacetamido]-3-(2-(2-thienyl)-3H-imidazolo-[4,5-b]pyridinium-4-ylmethyl)-3-cephem-4-carboxylate, and

7-[2-(5-amino-1,2,4-thiadiazol-3-yl)-2-methoxyiminoacetamido]-3-(2-aminooxazolo[5,4-c]pyridinium-5-ylmethyl)-3-cephem-4-carboxylate.

A preferred group of compounds are those represented by Formula (1) in which X is $N-R^2$ or S. Preferred compounds of the invention also are represented by Formula (1) in which R is an acyl group and R' is 2-aminothiazol-4-yl and R'' is $C_1-C_4$ alkyl, preferably methyl, or a carboxy-substituted alkyl group, preferably 2-carboxyprop-2-yl, 2-carboxymethyl, or 2-carboxyethyl.

The following non-limiting examples are provided to further illustrate the invention.

In the Examples, TMSI is trimethylsilyliodide; THF is tetrahydrofuran; HPLC is high performance liquid chromatography; NMR is nuclear magnetic resonance spectrum; DMSO-$d_6$ is deuterated dimethylsulfoxide; and the letters characterizing the NMR signals are as follows: s is singlet, d is doublet, q is quartet, m is multiplet, t is triplet, v is very, and b is broad. The NMR spectra were run on a JEOL FX-90.

## Preparation 1

3H-Imidazolo[4,5-c]pyridine was prepared by the method of Stanovik and Tisler, Synthesis, 2, 120

(1974). A mixture of 2.2 g (0.02 mole) of 3,4-diaminopyridine and 5 ml of diethoxymethyl acetate was heated at reflux for two hours. The reaction mixture was cooled and diluted by addition of ethyl acetate. The solid precipitate was collected by filtration and sublimed at 170°C (50 torr) to give 0.84 g of 3H-imidazolo[4,5-c]pyridine; mp 165-168°C.

Analysis calc. for $C_6H_5N_3$

Theory: C, 60.50; H, 4.23; N, 35.27.

Found: C, 60.15; H, 4.32; N, 34.94.

## Preparations 2-4

Following the general procedure of Preparation 1, the following imidazolopyridines were prepared:

1H-imidazolo[4,5-c]pyridine; mp 164-166°C.

3-methyl-3H-imidazolo[4,5-c]pyridine; mp 82-88°C.

1-methyl-1H-imidazolo[4,5-c]pyridine; mp 80°C.

## Preparation 5

A mixture 15.0 g of 3,4-diaminopyridine and 100 ml of acetic anhydride was heated at 120°C for seventy hours. The reaction mixture was cooled, concentrated and made alkaline to pH 11 by addition of 5N sodium hydroxide. The alkaline solution was extracted with chloroform and the extracts were combined, dried, and concentrated to dryness to give 5.8 g of 1H-2-methyl-imidazolo[4,5-c]pyridine; mp 164-166°C.

## Preparations 6-7

Similarly prepared were:

1,2-Dimethyl-1H-imidazolo[4,5-c]pyridine; mp 171-173°C.

2,3-Dimethyl-3H-imidazolo[4,5-c]pyridine; $M^+$ Theory 147; Found 147.

## Preparation 8-9

A mixture of 1.1 g (10 mM) of 3,4-diaminopyridine, 1.3 g (10 mM) of thiophene-2-carboxylic acid, and 50 g of polyphosphoric acid was heated at 160°C for four hours. The reaction mixture was added to 100 g of ice and stirred for fifteen minutes. The precipitated solid was collected by filtration and dried to give 500 mg of 2-(2-thienyl)-1H-imidazolo[4,5-c]pyridine; mp 265-268°C.

Similarly prepared was 2-phenyl-1H-imidazolo-[4,5-c]pyridine; 730 mg, single spot tlc (silica, chloroform-methanol; 90:10 v/v).

## Preparation 10

A mixture of 20 g (0.18 mole) of 2-amino-3-hydroxypyridine in 80 ml of water containing 20 g (0.19 mole) of cyanogen bromide was heated at reflux for fifteen minutes. The reaction mixture was filtered and the filtrate was cooled, neutralized by addition of sodium bicarbonate, and the precipitate that formed was collected by filtration and dried to give, following

recrystallization from ethanol and water, 8.66 g of 2-aminooxazolo[4,5-b]pyridine; mp 220-222°C.

Preparation 11

3-Amino-4-hydroxypyridine was reacted with acetic anhydride to afford 4.7 g of 2-methyloxazolo[4,5-c]pyridine; mp 56-58°C.

Preparation 12

Following the procedure of Takahashi, Chem. Pharm. Bull. (Tokyo) 2, (1954), 963 mg of 3-nitropyridine-4-thiol was reacted with 28.89 g of formic acid and 6.42 g of iron filings to provide, following purification over a silica gel column, 860 mg of thiazolo-[4,5-c]pyridine; mp 101-104°C.

Preparation 13

A mixture of 1.3 g of 3-nitropyridine-4-thiol in 4 ml of acetic acid and 15 ml of acetic anhydride containing 1.5 g of zinc dust was heated at reflux for four hours. The reaction mixture was cooled and concentrated to an oil. The oil was dissolved in 5N sodium hydroxide and the alkaline solution was extracted with diethyl ether. The extracts were combined, dried and concentrated to dryness to afford 557 mg of 2-methylthiazolo[4,5-c]pyridine. $M^+$ Theory 150; Found 150.

Preparation 14

3-Nitropyridine-4-thiol was reacted with propionic acid, propionic anhydride and zinc to give 2-ethylthiazolo[4,5-c]pyridine; mp 35°C.

Preparation 15

According to the method described in J. Het. Chem., 14(1), 129(1977) 2-chloro-3-aminopyridine was reacted with potassium thiocyanate and hydrochloric acid in ethanol to produce 45.3 g of 2-aminothiazolo[5,4-b]-pyridine. $M^+$ Theory 151; Found 151.

Example 1

*syn*-7-[2-(2-Aminothiazol-4-yl)-2-(2-carboxyprop-2-yl)oxyiminoacetamido]-3-(1H-imidazolo[4,5-c]-pyridinium-5-ylmethyl)-3-cephem-4-carboxylate

To a suspension of 1.34 g (2.5 mM) of *syn*-7-[2-(2-aminothiazol-4-yl)-2-(*tert*-butoxycarbonylprop-2-yl)oxyiminoacetamido]-3-acetoxymethyl-3-cephem-4-carboxylic acid in 15 ml of dichloromethane were added in one portion 1.42 ml (8 mM) of N-methyl-N-trimethylsilyltrifluoroacetamide. The reaction mixture was stirred for five minutes at 25°C under nitrogen. To the stirred solution was added by pipette 0.88 ml (6.2 mM) of TMSI and the reaction mixture then was stirred at 25°C for thirty minutes. The solvent was removed by evaporation under reduced pressure to provide an oil. The oil was

dissolved in 6 ml of acetonitrile and 0.84 ml (10.3 mM) of tetrahydrofuran and the solution was stirred for five minutes, whereupon a solution of 325 mg (2.7 mM) of 1H-imidazolo[4,5-c]pyridine (from Preparation 2) in 2 ml of acetonitrile containing 1 ml of N-methyl-N-trimethylsilyltrifluoroacetamide was added in one portion. The reaction mixture was stirred for three hours at 25°C and then added to a mixture of 60 ml of diethyl ether, 35 ml of acetone and 5 ml of methanol. The precipitated solid was collected by filtration to provide 630 mg (23% yield) of the product as a solid. The solid was purified by reverse phase $C_{18}$ silica HPLC using acetonitrile-acetic acid-water (10-2-88% by volume) as eluant. Removal of the solvents from the appropriate fractions afforded 120 mg of syn-7-[2-(2-aminothiazol-4-yl)-2-carboxyprop-2-yl)oxyiminoacetamido]-3-(1H-imidazolo[4,5-c]pyridinium-5-ylmethyl)-3-cephem-4-carboxylate.
IR(KBr): 1776 $cm^{-1}$ β-lactam;
UV (EtOH) $\lambda_{max}$ 220 ε 37,000;
$M^+$ Theory 586; Found 586;
NMR (DMSO-$d_6$): signals at 9.75 (s, 1H) δ 9.5 (d, 1H); 8.1 (d, 1H); 8.7 (d, 1H); 7.1 (bs, 2H); 6.7 (s, 1H); 5.7 (m, 1H); 5.15 (d, 1H); 1.4 (s, 6H);

## Example 2

syn-7-[2-(2-Aminothiazol-4-yl)-2-methoxyiminoacetamido]-3-(1H-imidazolo[4,5-c]pyridinium-5-ylmethyl)-3-cephem-4-carboxylate

A suspension of 910 mg (2 mM) of syn-7-[2-(2-aminothiazol-4-yl)-2-methoxyiminoacetamido]-3-acetoxymethyl-3-cephem-4-carboxylic acid in 5 ml of dichloromethane containing 1.24 ml (7 mM) of N-methyl-N-trimethylsilyltrifluoroacetamide was warmed to 40°C and sonicated for five minutes. The reaction mixture was cooled to 25°C and stirred while 0.77 ml (5.4 mM) of TMSI were added, and then stirring was continued at 25°C for thirty minutes. The solvent was removed by evaporation under reduced pressure and the oil was dissolved in 3 ml of acetonitrile and 0.77 ml (9 mM) of tetrahydrofuran. To this reaction mixture was added a solution of 297 mg (2.5 mM) of 1H-imidazolo[4,5-c]pyridine in 12 ml of acetonitrile containing 1.5 ml of N-methyl-N-trimethylsilyltrifluoroacetamide. The reaction mixture was stirred at 25°C for three hours and then added to 50 ml of 95% acetone-methanol (v/v). The precipitated solid was collected by filtration (yield 1.09 g) and purified by reverse phase $C_{18}$ silica HPLC using acetonitrile-acetic acid-water (4-2-94 percent by volume). There were obtained 390 mg of syn-7-[2-(2-aminothiazol-4-yl)-2-methoxyiminoacetamido]-3-(1H-imidazolo[4,5-c]pyridinium-5-ylmethyl)-3-cephem-4-carboxylate.

IR(KBr): 1772 $cm^{-1}$ β-lactam;
UV (EtOH) $\lambda_{max}$ 212 ε 34,000;
$M^+$ Theory 515; Found 515;
NMR (DMSO-$d_6$): signals at δ 9.85 (s, 1H); 9.55 (d, 1H); 8.9 (d, 1H); 8.8 (s, 1H); 8.15 (d, 1H); 7.2 (bs, 2H); 6.7 (s, 1H); 5.7 (m, 1H); 5.15 (d, 1H); 3.8 (s, 3H).

## Example 3

*syn*-7-[2-(2-Aminothiazol-4-yl)-2-methoxyimino-acetamido]-3-(3-methyl-3H-imidazolo[4,5-c]pyridinium-5-ylmethyl)-3-cephem-4-carboxylate was prepared according to the procedure of Example 2 by reacting 910 mg (2 mM) of *syn*-7-[2-(2-aminothiazol-4-yl)-2-methoxyiminoacetamido]-3-acetoxymethyl 4-carboxylic acid with 1.24 ml (7.0 mM) of N-methyl-N-trimethylsilyltrifluoroacetamide and 0.77 ml (5.4 mM) of TMSI to produce the corresponding 3-iodomethyl cephalosporin, and reacting the latter compound *in situ* with 3-methyl-3H-imidazolo[4,5-c]pyridine. The product was obtained as 920 mg of a white solid. Purification over $C_{18}$ reverse phase HPLC gave 340 mg of title compound.
IR(KBr): 1772 $cm^{-1}$;
UV (EtOH) $\lambda_{max}$ 210 ε 36,500;
$M^+$ Theory 529; Found 529;
NMR (DMSO-$d_6$): signals at w 9.5 (d, 1H) 9.4 (d, 1H); 9.05 (s, 1H); 8.35 (d, 1H), 7.2 (bs, 2H); 6.73 (s, 1H); 5.75 (m, 1H), 5.15 (d, 1H); 4.15 (s, 3H), 3.83 (s, 3H).

## Examples 4-16

The following 3-bicyclicpyridiniummethyl cephalosporins were prepared by the methods of Examples 1-3 by reacting a bicyclic pyridine with a 3-iodomethyl cephalosporin derived from the corresponding 3-acetoxymethyl cephalosporin derivative:

## Example 4

syn-7-[2-(2-aminothiazol-4-yl)-2-methoxyiminoacetamido]-3-(1-methyl-1H-imidazolo[4,5-c]pyridinium-5-ylmethyl)-3-cephem-4-carboxylate. Yield 86%
IR(KBr): 1773 $cm^{-1}$ β-lactam;
$M^+$ Theory 529; Found 529.
NMR (DMSO-$d_6$): signals at δ 10.1 (s, 1H), 9.45 (d, 1H); 9.1 (d, 1H), 8.8 (s, 1H), 8.3 (d, 1H), 7.15 (bs, 2H), 6.65 (s, 1H), 5.65 (m, 1H), 5.05 (m, 1H), 4.0 (s, 3H), 3.75 (s, 3H).

## Example 5

syn-7-[2-(2-aminothiazol-4-yl)-2-methoxyiminoacetamido]-3-(2-methyl-1H-imidazolo[4,5-c]pyridinium-5-ylmethyl)-3-cephem-4-carboxylate. Yield 96%.
NMR (DMSO-$d_6$): signals at δ 9.9 (s, 1H), 9.5 (d, 1H), 8.85 (d, 1H); 8.05 (d, 1H), 7.1 (bs, 2H), 6.66 (s, 1H), 5.7 (m, 1H), 5.1 (d, 1H), 3.75 (s, 3H) 2.7 (s, 3H).

## Example 6

syn-7-[2-(2-aminothiazol-4-yl)-2-methoxyiminoacetamido]-3-(1,2-dimethyl-1H-imidazolo[4,5-c]pyridinium-5-ylmethyl)-3-cephem-4-carboxylate. Yield 80%
IR(KBr): 1774 $cm^{-1}$ β-lactam;
UV (EtOH) $\lambda_{max}$ 218 ε 48,500.

NMR (DMSO-$d_6$): signals at δ 9.8 (s, 1H), 9.5 (d, 1H), 9.1 (d, 1H), 8.2 (d, 1H), 7.15 (bs, 2H), 6.65 (s, 1H), 5.6 (m, 1H), 5.05 (m, 1H); 3.9 (s, 3H), 3.75 (s, 3H), 2.7 (s, 3H).

## Example 7

syn-7-[2-(2-aminothiazol-4-yl)-2-methoxyiminoacetamido]-3-(2,3-dimethyl-3H-imidazolo[4,5-c]-pyridinium-5-ylmethyl)-3-cephem-4-carboxylate. Yield 53%

IR(KBr): 1775 $cm^{-1}$ β-lactam;

UV (EtOH) $\lambda_{max}$ 206 ε 38,000;

$M^+$ Theory 543; Found 543.

NMR (DMSO-$d_6$): signals at δ 9.9 (s, 1H), 9.5 (d, 1H), 9.3 (d, 1H), 8.1 (d, 1H), 7.15 (bs, 2H), 6.7 (s, 1H), 5.7 (m, 1H), 5.1 (d, 1H), 3.95 (s, 3H), 3.8 (s, 3H), 2.8 (s, 3H).

## Example 8

syn-7-[2-(2-aminothiazol-4-yl)-2-methoxyiminoacetamido]-3-[2-(2-thienyl)-1H-imidazolo[4,5-c]-pyridinium-5-ylmethyl)-3-cephem-4-carboxylate. Yield 18.7%

IR(KBr): 1774 $cm^{-1}$ β-lactam;

UV (EtOH) $\lambda_{max}$ 247 ε 25,500;

$M^+$ Theory 596; Found 597.

NMR (DMSO-$d_6$): signals at δ 9.5 (d, 1H), 9.3 (s, 1H), 8.5 (d, 3H), 7.8 (m, 2H), 7.2 (bs, 2H), 6.7 (s, 1H), 5.7 (m, 1H), 5.1 (d, 1H), 3.8 (s, 3H).

## Example 9

<u>syn</u>-7-[2-(2-aminothiazol-4-yl)-2-methoxyimino-acetamido]-3-(2-phenyl-1H-imidazolo[4,5-c]pyridinium-5-ylmethyl)-3-cephem-4-carboxylate. Yield 620 mg.
IR(KBr): 1772 $cm^{-1}$ β-lactam;
UV (EtOH) $\lambda_{max}$ 242 ε 39,500;
$M^+$ Theory 591; Found 591.
NMR (DMSO-$d_6$): signals at δ 9.5-7.5 (m, 8H), 7.1 (s, 2H), 6.7 (s, 1H), 5.7 (m, 1H), 5.15 (d, 1H), 3.8 (s, 3H).

## Example 10

<u>syn</u>-7-[2-(2-aminothiazol-4-yl)-2-methoxy-iminoacetamido]-3-(2-aminooxazolo[4,5-b]pyridinium-4-ylmethyl)-3-cephem-4-carboxylate. Yield 100%
IR(KBr): 1772 $cm^{-1}$ β-lactam;
1695
1656
UV (EtOH) $\lambda_{max}$ 205, 235, 315 ε 22,746.
NMR (DMSO-$d_6$): signals at δ 9.5 (d, 1H), 9.05 (d, 1H), 8.1 (d, 1H), 7.33 (d, 1H), 7.1 (bs, 2H), 6.7 (s, 1H), 5.6 (m, 1H), 5.0 (d, 1H), 3.8 (s, 3H).

Example 11

syn-7-[2-(2-aminothiazol-4-yl)-2-methoxyiminoacetamido]-3-(2-methyloxazolo[4,5-c]pyridinium-5-ylmethyl)-3-cephem-4-carboxylate. Yield 74%
IR(KBr): 1776 $cm^{-1}$ β-lactam;
UV (EtOH) $\lambda_{max}$ 203 ε 41,500;
$M^+$ Theory 530; Found 530.
NMR (DMSO-$d_6$): signals at δ 10.2 (s, 1H), 9.5 (m, 2H), 8.5 (d, 1H), 5.6 (m, 1H), 5.0 (d, 1H), 3.8 (s, 3H), 2.8 (s, 3H).

Example 12

syn-7-[2-(2-aminothiazol-4-yl)-2-methoxyiminoacetamido]-3-(thiazolo[4,5-c]pyridinium-5-ylmethyl)-3-cephem-4-carboxylate. Yield 95%
IR(KBr): 1773 $cm^{-1}$ β-lactam;
NMR (DMSO-$d_6$): signals at δ 10.4 and 9.9 (d, 4H), 7.7 (s, 1H), 6.1 (d, 1H), 3.8 (s, 3H).

Example 13

syn-7-[2-(2-aminothiazol-4-yl)-2-methoxyiminoacetamido]-3-(2-methylthiazolo[4,5-c]pyridinium-5-ylmethyl)-3-cephem-4-carboxylate. Yield 48%
IR(KBr): 1777 $cm^{-1}$ β-lactam;
1674
1623
UV (EtOH) $\lambda_{max}$ 227, 260 ε 21,975;

NMR (DMSO-$d_6$): signals at δ 10.15 (s, 1H), 9.5 (d, 1H), 9.25 (d, 1H), 8.75 (d, 1H), 5.6 (m, 1H), 5.0 (d, 1H), 3.8 (s, 3H), 2.95 (s, 3H).

Example 14

*syn*-7-[2-(2-aminothiazol-4-yl)-2-methoxyimino-acetamido]-3-(2-ethylthiazolo[4,5-c]pyridinium-5-yl-methyl)-3-cephem-4-carboxylate.
IR(KBr): 1772 $cm^{-1}$ β-lactam;
UV (EtOH) $\lambda_{max}$ 230 ε 46,500;
$M^+$ Theory 559; Found 560.
NMR (DMSO-$d_6$): signals at δ 10.15 (s, 1H), 9.35 (d, 1H), 8.8 (d, 1H), 5.6 (m, 1H), 5.0 (d, 1H), 3.8 (s, 3H), 3.3 (q, 2H), 1.4 (t, 3H).

Example 15

*syn*-7-[2-(2-aminothiazol-4-yl)-2-methoxyimino-acetamido]-3-(2-aminothiazolo[5,4-b]pyridinium-4-yl-methyl)-3-cephem-4-carboxylate. Yield 100%
UV (EtOH) $\lambda_{max}$ 250 ε 25,633;
Analysis calculated for $C_{20}H_{18}N_8O_5S_3$
Theory: C, 43.95; H, 3.32; N, 20.50; S, 17.60.
Found: C, 42.07; H, 3.80; N, 17.84; S, 15.71.
NMR (DMSO-$d_6$): signals at δ 9.5 (d, 1H), 8.75 (m, 3H), 8.1 (d, 1H), 7.73 (m, 1H), 5.6 (m, 1H), 5.0 (d, 1H), 3.8 (s, 3H).
Titration (66% dimethylformamide in water v/v) $pK_a$ at 4.0, 7.4, 10.7.

Example 16

*syn*-7-[2-(2-aminothiazolo-4-yl)-2-methoxyimino-acetamido]-3-(2-methylthiazolo[5,4-c]pyridinium-5-yl-methyl)-3-cephem-4-carboxylate.

UV (EtOH) $\lambda_{max}$ 258 $\varepsilon$ 20,563;

IR (KBr) 1774 $cm^{-1}$ ($\beta$-lactam);

$M^+$ Theory 546; Found 546.

The 3-bicyclicpyridinium methyl cephalosporins provided by this invention are useful as antibiotic substances. The compounds have demonstrated excellent antibacterial activity against a wide variety of Gram + and Gram - bacilli. The compounds are particularly effective against diseases caused by Steptococci, H. influenza, E. coli, Klebsiella, Enterobacter, Salmonella, and Serratia.

The antibacterial activity of several representative compounds of the invention has been evaluated in standard *in vitro* agar dilution assays. The following Table presents typical minimum inhibitory concentrations (MIC's) in μg/ml for exemplary compounds when evaluated against several Gram + and Gram - microorganisms. The activity of the known compound, ceftazidime, is given for comparison.

Table I

Agar Dilution MIC (μg/ml)

| Organism | Strain | Ceftazidime | Compound of Example No. 1 | 2 | 3 | 4 | 5 | 6 | 7 |
|---|---|---|---|---|---|---|---|---|---|
| Staph. aureus | X1.1 | 8 | 8 | 1 | 1 | 1 | 2 | 2 | 2 |
| | V41 | 32 | 32 | 8 | 4 | 8 | 8 | 16 | 16 |
| | X400 | 128 | 128 | 64 | 64 | 64 | 32 | 32 | 128 |
| | S13E | 32 | 32 | 8 | 4 | 8 | 8 | 8 | 16 |
| Staph. epi | EPI1 | 16 | 32 | 4 | 2 | 2 | 4 | 2 | 4 |
| | 222 | 16 | 16 | 1 | 1 | 1 | 1 | 1 | 2 |
| Strep. A | C203 | 0.125 | 0.06 | <0.008 | <0.008 | 0.15 | <0.008 | <0.008 | 0.015 |
| Strep. PN | PARK | 0.125 | 0.06 | <0.008 | <0.008 | 0.15 | <0.008 | <0.008 | - |
| Strep. D | X66 | >128 | >128 | 128 | >128 | >128 | >128 | >128 | >128 |
| | 9960 | 16 | 16 | 2 | 4 | 2 | 8 | 4 | 4 |
| H. influ. | C.L. | 0.125 | 0.03 | 0.015 | 0.06 | 0.06 | 0.015 | 0.06 | 0.06 |
| | 76 | 0.125 | 0.06 | 0.015 | 0.06 | 0.06 | 0.015 | - | 0.03 |
| E. coli | N10 | 0.25 | 0.5 | 0.015 | 0.06 | 0.03 | 0.03 | 0.03 | 0.03 |
| | EC14 | 0.125 | 0.125 | <0.008 | <0.008 | <0.008 | <0.008 | <0.008 | 0.015 |
| | TEM | 0.125 | 0.125 | <0.008 | <0.008 | 0.015 | <0.008 | <0.008 | 0.015 |
| Klebsiella | X26 | 0.06 | 0.125 | <0.008 | <0.008 | 0.015 | <0.008 | <0.008 | 0.015 |
| | KAE | 0.5 | 1.0 | 4 | 4 | 4 | 2 | 2 | 2 |
| | X68 | 0.125 | 0.125 | <0.008 | 0.03 | 0.015 | 0.015 | 0.03 | 0.03 |
| Enterobacter | | | | | | | | | |
| aerogenes | C32 | 0.25 | 1.0 | 0.03 | 0.06 | 0.03 | 0.03 | 0.03 | |
| cloacae | EB5 | 0.125 | 0.25 | 0.06 | 0.06 | 0.03 | 0.06 | 0.03 | |
| Salmonella | X514 | 0.125 | 0.5 | <0.008 | <0.008 | 0.05 | <0.008 | 0.03 | |
| Pseudomonas | X528 | 2 | 8 | 1.0 | 1 | 1 | 2 | 4 | |
| | X239 | 2 | 2 | 2 | 2 | 2 | 2 | 4 | |
| Serratia | X99 | 0.25 | 0.5 | 0.015 | 0.06 | 0.06 | 0.03 | 0.03 | |

Table I (cont'd)

Agar Dilution MIC (μg/ml)

| Organism | Strain | 8 | 9 | 10 | 11 | 12 | 13 | 14 | 15 |
|---|---|---|---|---|---|---|---|---|---|
| | | Compound of Example No. | | | | | | | |
| Staph. aureus | X1.1 | 2 | 2 | | 0.5 | 1 | 1 | 1 | |
| | V41 | 8 | 8 | 4 | 8 | 2 | 8 | 4 | 2 |
| | X400 | 32 | 32 | 32 | 32 | 2 | 32 | 32 | 32 |
| | S13E | 8 | 8 | 4 | 8 | 8 | 8 | 4 | 4 |
| Staph. epi | EPI1 | 4 | 4 | 2 | 4 | 2 | 2 | 2 | 2 |
| | 222 | 2 | 2 | 1 | 1 | - | 1 | 1 | 16 |
| Strep. A | C203 | <0.008 | <0.008 | 0.015 | 0.015 | 0.015 | 0.015 | <0.008 | 0.015 |
| Strep. PN | PARK | <0.008 | <0.008 | 0.015 | 0.015 | 0.015 | <0.008 | <0.008 | - |
| Strep. D | X66 | >128 | >128 | >128 | >128 | 128 | >128 | >128 | 128 |
| | 9960 | 4 | 4 | 16 | 8 | 4 | 8 | 4 | 8 |
| H. influ. | C.L. | 0.25 | 0.5 | 0.06 | 0.125 | 0.06 | 0.03 | 0.06 | 0.06 |
| | 76 | - | - | 0.125 | 0.06 | 0.06 | 0.03 | 0.015 | 0.06 |
| E. coli | N10 | 1 | 1 | 0.125 | 0.06 | 0.03 | 0.03 | 0.03 | 0.03 |
| | EC14 | 0.25 | 0.125 | 0.06 | 0.03 | 0.015 | 0.015 | 0.015 | 0.06 |
| | TEM | 0.03 | 0.06 | 0.03 | 0.03 | 0.03 | 0.015 | 0.015 | 0.03 |
| Klebsiella | X26 | 0.015 | 0.015 | 0.03 | 0.015 | 0.015 | 0.015 | <0.008 | 0.03 |
| | KAE | 4 | 2 | 4 | 4 | 4 | 2 | 2 | - |
| | X68 | 0.5 | 0.5 | 0.06 | 0.06 | 0.03 | 0.03 | 0.03 | 4 |
| Enterobacter | | | | | | | | | |
| aerogenes | C32 | 0.5 | 0.5 | 0.125 | 0.06 | 0.03 | 0.03 | 0.06 | 0.03 |
| cloacae | EB5 | 0.5 | 0.5 | 0.25 | 0.125 | 0.06 | 0.06 | 0.06 | 0.06 |
| Salmonella | X514 | 0.5 | 0.5 | 0.06 | 0.03 | 0.03 | 0.06 | 0.06 | 0.03 |
| Pseudomonas | X528 | 64 | 64 | 4 | 4 | 1 | 2 | 4 | 1 |
| | X239 | 64 | 64 | 2 | 2 | 2 | 8 | 4 | 2 |
| Serratia | X99 | 0.5 | 1.0 | 0.125 | 0.125 | 0.06 | 0.06 | 0.06 | 0.06 |

The excellent antibacterial activity of the compounds provided by this invention make them particularly attractive agents for the treatment of a number of diseases of bacterial origin. The treatment of animals suffering from bacterial diseases, or suspected of developing a bacterial infection, is thus another embodiment of this invention. The antibacterial method of treatment provided by this invention is practiced by administering an antibacterially-effective amount of a 3-bicyclicpyridinium methyl cephalosporin antibiotic as defined herein to an animal in need of treatment. The method can be practiced therapeutically or prophylactically. The amount of active antibiotic to be administered according to the method will vary depending upon the particular compound selected, the severity of the disease being treated or guarded against, the individual undergoing treatment, and related factors commonly encountered with such treatments. Normally, however, the compounds will be administered at a dose of about 0.5 to about 50 mg/kg of animal body weight, and more preferably at a rate of about 1 to about 10 mg/kg. Such amounts may be administered once each day, or more often as needed to treat the particular disease or subject undergoing treatment according to the present method. A typical daily dose for an average adult human will be about 200 to about 500 mg per day.

The antibiotic compounds provided by this invention are especially active when administered by the parenteral route, but they can be formulated for any desired route of administration. Such formulations

constitute yet another embodiment of this invention. The formulations of this invention will comprise from about 0.1 to about 95 percent by weight of an active cephalosporin antibiotic of the invention (compounds of Formula (1) in which R is acyl), admixed with a pharmaceutically-acceptable carrier, diluent or excipient therefor. Typical formulations may contain from about 10 to about 60 percent by weight of active ingredient, and more preferably about 20 to about 50 percent.

For convenient oral administration, the compounds can be admixed with any of a number of diluents, excipients and carriers commonly employed in oral formulations, and molded into tablets, pills, troches, or encapsulated into gelatin capsules. Typical carriers, diluents and excipients commonly employed include potato starch, corn starch, sucrose, dextrose, microcrystalline cellulose, dicalcium phosphate, alginic acid, acacia; lubricants such as magnesium stearate; binders such as gum tragacanth or gelatin; and flavoring agents such as peppermint oil, cherry or strawberry flavoring, or oil of wintergreen. The compounds also can be formulated as syrups or elixirs employing common diluents such as a fatty oil, methyl or propyl parabens, suitable dyes and flavoring agents. The compounds also can be formulated in the form of a buccal seal, lozenge or other suitable device for sustained controlled delivery of the active ingredient over a prolonged period.

The antibiotics of the invention preferably are formulated for parenteral administration, for

example via the intravenous, intramuscular or subcutaneous routes, as well as the transdermal route. Such compositions normally will contain from about 0.1 to about 20.0 percent by weight of active ingredient. Typical excipients, diluents and carriers for parenteral formulations include isotonic saline, dilute aqueous dextrose (e.g. 5%), the polyhydric aliphatic alcohols or mixtures thereof, for instance, glycerin, propylene glycol, or polyethylene glycol. Parenteral solutions also may contain preservatives such as phenethylalcohol, methyl and propyl parabens, and thimerosal. If needed, about 0.05 to about 0.20 percent by weight of an antioxidant such as sodium metabisulfite or sodium bisulfite also can be employed. For intravenous use, preferred formulations will employ an initial concentration down to about 0.05 to about 0.25 mg/ml of active ingredient, and for intramuscular injection, a preferred concentration of active ingredient is about 0.25 to about 0.50 mg/ml.

Examples of typical pharmaceutical formulations may include the following.

Example 17

Formulation for Intravenous Use

| Ingredient | Amount |
|---|---|
| Compound of Example 2 | 1.0 g |
| 0.9% saline | 100 ml |

The intravenous solution can be prepared, for example, with a unit dosage formulation of the antibiotic in a plastic bag or similar container, and by adding the diluent to the container prior to infusion.

Example 18

Formulation of Oral Suspension

| Ingredient | Amount |
|---|---|
| Compound of Example 8 | 500 mg |
| Sorbitol solution (70% N.F.) | 40 ml |
| Sodium benzoate | 150 mg |
| Saccharin | 10 mg |
| Cherry flavor | 50 mg |
| Distilled water q s ad | 100 ml |

The sorbitol solution is added to 40 ml of distilled water and the cephalosporin is suspended thereon. The saccharin, sodium benzoate, and flavoring are added and dissolved. The volume is adjusted to 100 ml with distilled water. Each ml of syrup contains 5 mg of the cephalosporin antibiotic. This oral formulation is suited ideally for pediatric use.

Example 19

Preparation of 250 mg capsule

| Ingredient | Amount |
|---|---|
| Compound of Example 10 | 250 mg |

| | |
|---|---|
| Lactose | 150 mg |
| Corn starch | 100 mg |
| | 500 mg |

The ingredients are blended to uniformity and encapsulated into gelatin capsules. Such capsules may be administered orally at the rate of about one each day for the treatment of upper respiratory bacterial infections, including pharyngitis and tonsillitis.

Example 20

Preparation of Parenteral Solution

In a solution of 700 ml of propylene glycol and 200 ml of distilled water for injection is dissolved 20.0 grams of the compound of Example 1, as the hydrochloride salt. The pH of the solution is adjusted to 5.5 with hydrochloric acid, and the volume is made up to 1000 ml with distilled water. The formulation is sterilized, filled into 5.0 ml ampoules each containing 2.0 ml (representing 40 mg of active ingredient) and sealed under nitrogen.

The compounds of the invention additionally may be administered intrarectally, for example in a suitably formulated suppository. Pharmaceutically-acceptable suppository formulations can be prepared with the antibiotic compound and a suppository composition such as cocoa butter, hydrogenated fats, glycerides, or polyethylene glycols.

Pharmaceutical compositions of the invention also include unit dosage formulations. Such formulations comprise between about 200 mg. and about 10 g. of the antibiotic or a pharmaceutically-acceptable salt thereof in solid form in a sterile ampoule, vial or a plastic container such as a bag adapted for i.v. administration. The antibiotic may be amorphous or in the crystalline state. Such formulations may also contain a buffering agent, solubilizing agent, clarifying agent, stabilizing agent, or other excipient. An example of a pharmaceutical composition of this invention for i.v. use comprises 500 mg. of the dry powder of the antibiotic or a pharmaceutically acceptable salt thereof in a 10 ml. sterile rubber-stoppered ampoule. Another such composition comprises 4 g. of dry powder of the antibiotic in a 100 ml. sterile ampoule. A further composition comprises 10 g. of the antibiotic as a dry powder in a sealed, sterile plastic pouch.

CLAIMS

1. A compound of Formula (1):

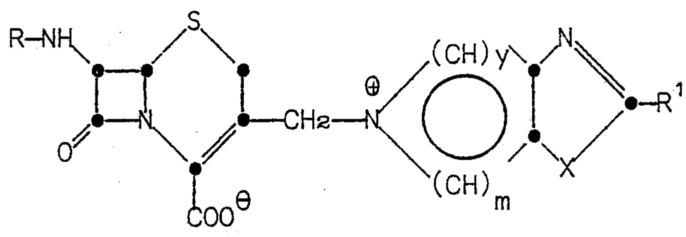 1

in which R is hydrogen, formyl, α-aminoadipoyl, protected α-aminoadipoyl, or an acyl group of the formula

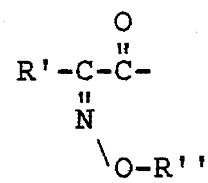

in which R' is a 5- or 6-membered heterocyclic ring of the formulae

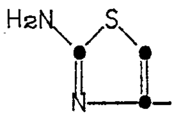 , 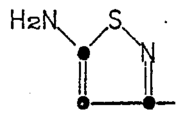 , 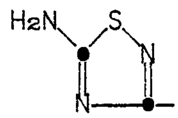 , 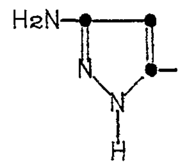 ,

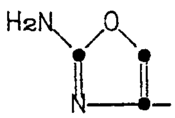 , 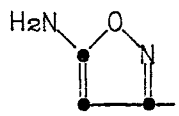 , 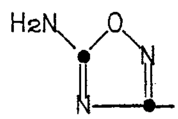 , 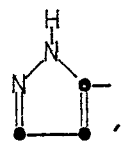 ,

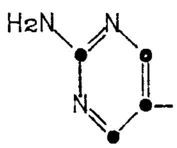 , 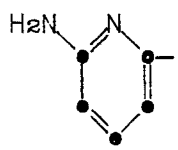 , or 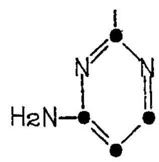 ;

R'' is hydrogen, $C_1$-$C_4$ alkyl, a carboxy-substituted alkyl or carboxy-substituted cycloalkyl group of the formula:

$$-\overset{a}{\underset{b}{C}}-(CH_2)_n-COR'''$$

in which n is 0-3; a and b when taken separately are, independently, hydrogen or $C_1$-$C_3$ alkyl, and when taken together with the carbon to which they are bonded form a $C_3$-$C_7$ carbocyclic ring; R''' is hydroxy, $C_1$-$C_4$ alkoxy, amino, or OR°, in which R° is indanyl, phthalidyl, or an acyloxymethyl group of the formula $-CH_2$-O-C(O)-$R_2$ in which $R_2$ is $C_1$-$C_4$ alkyl or phenyl; or COOR° is a protected carboxy group;

or R'' is an N-substituted carbamoyl group of the formula

O
"
-C-NHR''''

in which R'''' is $C_1$-$C_4$ alkyl, phenyl or $C_1$-$C_3$ alkyl substituted by phenyl;

y and m, independently, are integers equal to 0, 1, 2 or 3, provided that y plus m equals 3;

$R^1$ is hydrogen, $C_1$-$C_4$ alkyl, phenyl, thienyl, amino or $C_1$-$C_4$ alkanoylamino;

X is O, S or N-$R^2$, where $R^2$ is hydrogen or $C_1$-$C_4$ alkyl; or a pharmaceutically-acceptable salt or biologically-cleavable ester thereof.

2. A compound of Formula (1):

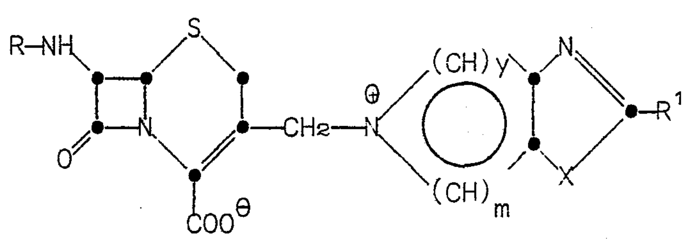 1

in which R is hydrogen, formyl, α-aminoadipoyl, protected α-aminoadipoyl, or an acyl group of the formula

O
"
R'-C-C-
"
N
\
O-R''

in which R' is a 5- or 6-membered heterocyclic ring of the formulae

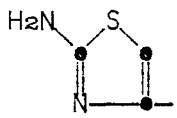 , 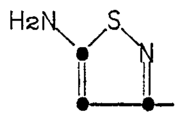 , 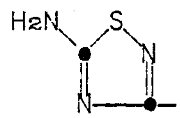 , 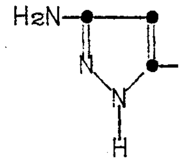 ,

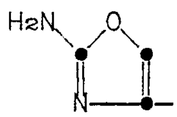 , 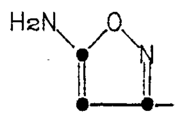 , 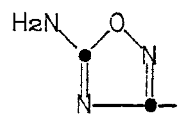 , 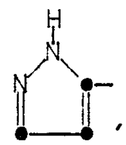 ,

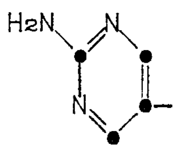 , 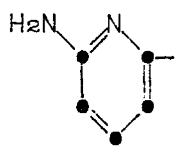 , or 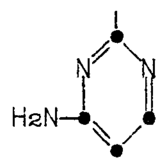 ;

R'' is hydrogen, $C_1$-$C_4$ alkyl, a carboxy-substituted alkyl or carboxy-substituted cycloalkyl group of the formula:

$$-\overset{a}{\underset{b}{C}}-(CH_2)_n-COR'''$$

in which n is 0-3; a and b when taken separately are, independently, hydrogen or $C_1$-$C_3$ alkyl, and when taken together with the carbon to which they are bonded form a $C_3$-$C_7$ carbocyclic ring; R''' is hydroxy, $C_1$-$C_4$ alkoxy, amino, or OR°, in which R° is indanyl, phthalidyl, or an acyloxymethyl group of the formula $-CH_2-O-C(O)-R_2$ in which $R_2$ is $C_1$-$C_4$ alkyl or phenyl; or COOR° is a protected carboxy group;

or R'' is an N-substituted carbamoyl group of the formula

$$-\overset{O}{\overset{\|}{C}}-NHR''''$$

in which R'''' is $C_1$-$C_4$ alkyl, phenyl or $C_1$-$C_3$ alkyl substituted by phenyl;

y and m, independently, are integers equal to 0, 1, 2 or 3, provided that y plus m equals 3;

$R^1$ is hydrogen, $C_1$-$C_4$ alkyl, phenyl, thienyl, amino or $C_1$-$C_4$ alkanoylamino;

X is O, S or N-$R^2$, where $R^2$ is hydrogen or $C_1$-$C_4$ alkyl; or a pharmaceutically-acceptable salt or biologically-cleavable ester thereof, provided that when R' is

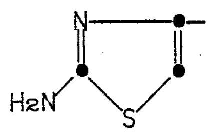 ;

and R'' is $C_1$-$C_4$ alkyl, or if n=0, and a and b, independently, are hydrogen, methyl, ethyl or when a and b are taken together with the carbon to which they are attached form a $C_3$-$C_5$ carbocyclic ring; and y=1 and m=2; and X=S, then $R^1$ may only be phenyl, thienyl or $C_1$-$C_4$ alkanoylamino.

3. A compound of Formula (1), or a pharmaceutically-acceptable salt or ester thereof, as claimed in claim 1 or 2 in which R' is 2-amino-thiazol-4-yl.

4. A compound of Formula (1), or a pharmaceutically-acceptable salt or ester thereof, as claimed in claim 3 in which y is 1 and m is 2.

5. A compound of Formula (1), as claimed in any one of claims 1 to 4 in which X is N-$R^2$ or O.

6. A compound of Formula (1), as claimed in any one of claims 1 to 4 in which X is S.

7. syn-7-[2-(2-Aminothiazol-4-yl)-2-(2-carboxyprop-2-yl)oxyiminoacetamido]-3-(1H-imidazolo[4,5-c]-pyridinium-5-ylmethyl)-3-cephem-4-carboxylate

syn-7-[2-(2-Aminothiazol-4-yl)-2-methoxyiminoacetamido]-3-(1H-imidazolo]4,5-c]pyridinium-5-ylmethyl)-3-cephem-4-carboxylate

syn-7-[2-(2-Aminothiazol-4-yl)-2-methoxyiminoacetamido]-3-(3-methyl-3H-imidazolo[4,5-c]pyridinium-5-ylmethyl)-3-cephem-4-carboxylate

syn-7-[2-(2-Aminothiazol-4-yl)-2-methoxyiminoacetamido]-3-(1-H-imidazolo[4,5-c]pyridinium-5-ylmethyl)-3-cephem-4-carboxylate

syn-7-[2-(2-Aminothiazol-4-yl)-2-methoxyiminoacetamido]-3-(2-methyl-1H-imidazolo[4,5-c]pyridinium-5-ylmethyl)-3-cephem-4-carboxylate

syn-7-[2-(2-Aminothiazol-4-yl)-2-methoxyiminoacetamido]-3-(1,2-dimethyl-1H-imidazolo[4,5-c]pyridinium-5-ylmethyl)-3-cephem-4-carboxylate

syn-7-[2-(2-Aminothiazole-4-yl)-2-methoxyiminoacetamido]-3-(2,3-dimethyl-3H-imidazolo[4,5-c]-pyridinium-5-ylmethyl)-3-cephem-4-carboxylate

syn-7-[2-(2-Aminothiazol-4-yl)-2-methoxyiminoacetamido]-3-[2-(2-thienyl)-1H-imidazolo[4,5-c]-pyridinium-5-ylmethyl)-3-cephem-4-carboxylate

syn-7-[2-(2-Aminothiazole-4-yl)-2-methoxyiminoacetamido]-3-(2-phenyl-1H-imidazolo[4,5-b]-pyridinium-5-ylmethyl)-3-cephem-4-carboxylate

syn-7-[2-(2-Aminothiazol-4-yl)-2-methoxyiminoacetamido]-3-(2-aminooxazolo[4,5-b]-pyridinium-4-ylmethyl)-3-cephem-4-carboxylate

syn-7-[2-(2-Aminothiazol-4-yl)-2-methoxyiminoacetamido]-3-(2-methyloxazolo[4,5-c]-pyridinium-5-ylmethyl)-3-cephem-4-carboxylate

syn-7-[2-(2-Aminothiazol-4-yl)-2-methoxyiminoacetamido]-3-(2-aminothiazolo[5,4-b]-pyridinium-4-ylmethyl)-3-cephem-4-carboxylate

or a pharmaceutically-acceptable salt or biologically-cleavable ester thereof.

8. syn-7-[2-(2-Aminothiazol-4-yl)-2-methoxyiminoacetamido]-3-(thiazolo[4,5-c]-pyridinium-5-ylmethyl)-3-cephem-4-carboxylate

syn-7-[2-(2-Aminothiazol-4-yl)-2-methoxyiminoacetamido]-3-(2-methylthiazolo[4,5-c]pyridinium-5-ylmethyl)-3-cephem-4-carboxylate

syn-7-[2-(2-aminothiazolo-4-yl)-2-methoxyiminoacetamido]-3-(2-methylthiazolo[5,4-c]pyridinium-5-ylmethyl)-3-cephem-4-carboxylate.

syn-7-[2-(2-Aminothiazol-4-yl)-2-methoxyiminoacetamido]-3-(2-ethylthiazolo[4,5-c]pyridinium-5-ylmethyl)-3-cephem-4-carboxylate or a pharmaceutically-acceptable salt or biologically-cleavable ester thereof.

9. A process for preparing a cephalosporin derivative of Formula (1) or a pharmaceutically-acceptable salt or biologically-cleavable ester thereof, as claimed in any one of claims 1 to 8, which comprises:

(a) condensing a compound of Formula (2):

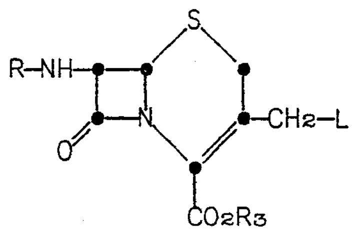 (2)

in which L is a leaving group, $R_3$ is hydrogen or a carboxy-protecting group, and R is as defined in claim 1 or 2; with a bicyclicpyridine compound of the Formula:

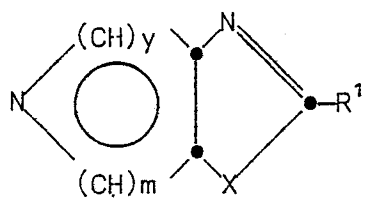 ,

and if desired, removing any protecting group which may be present, and/or salifying or esterifying the product.

(b) acylating a compound of Formula (1) in which R is hydrogen, or a salt or 4'-ester thereof, with an acid of the Formula:

R'-C-COOH
   ‖
   N-OR''

or an activated derivative thereof, and if desired, removing any protecting group present and/or salifying or esterifying the product.

(c) deacylating a compound of Formula (1) in which R is other than hydrogen, or a salt or

ester thereof to form a compound in which R is hydrogen, or a salt or ester thereof.

10. A compound of Formula (1), or a pharmaceutically-acceptable salt or biologically-cleavable ester thereof, as claimed in any one of claims 1 to 8 for use as an antibiotic.

11. A pharmaceutical formulation which comprises as an active ingredient, a compound of Formula (1), in which R is an acyl group, or a pharmaceutically-acceptable salt or biologically-cleavable ester thereof, as claimed in any one of claims 1 to 8, associated with one or more pharmaceutically-acceptable carriers, excipients or diluents therefor.

CLAIMS

1. A process for preparing a compound of Formula (1):

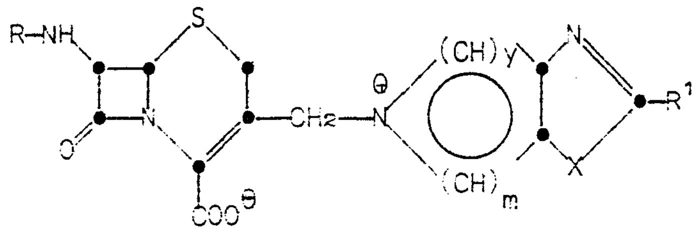

I

in which R is hydrogen, formyl, α-aminoadipoyl, protected α-aminoadipoyl, or an acyl group of the formula

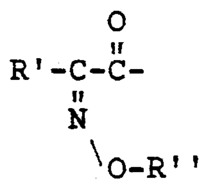

in which R' is a 5- or 6-membered heterocyclic ring of the formulae

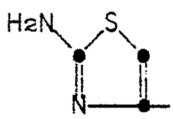 , 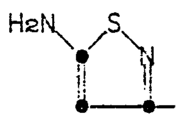 , 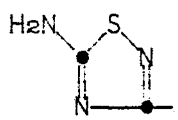 , 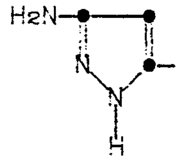 ,

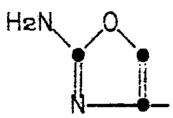 , 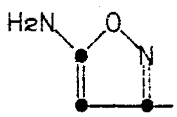 , 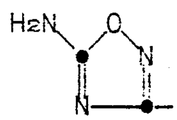 , 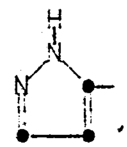 ,

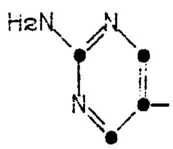 , 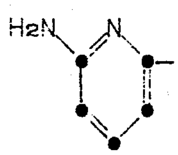 , or 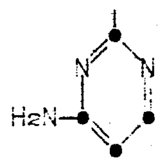 ;

R'' is hydrogen, $C_1$-$C_4$ alkyl, a carboxy-substituted alkyl or carboxy-substituted cycloalkyl group of the formula:

$$-\overset{a}{\underset{b}{C}}-(CH_2)_n-COR'''$$

in which n is 0-3; a and b when taken separately are, independently, hydrogen or $C_1$-$C_3$ alkyl, and when taken together with the carbon to which they are bonded form a $C_3$-$C_7$ carbocyclic ring; R''' is hydroxy, $C_1$-$C_4$ alkoxy, amino, or OR°, in which R° is indanyl, phthalidyl, or an acyloxymethyl group of the formula $-CH_2-O-C(O)-R_2$ in which $R_2$ is $C_1$-$C_4$ alkyl or phenyl; or COOR° is a protected carboxy group;

or R'' is an N-substituted carbamoyl group of the formula

$$-\overset{O}{\overset{\|}{C}}-NHR''''$$

in which R'''' is $C_1$-$C_4$ alkyl, phenyl or $C_1$-$C_3$ alkyl substituted by phenyl;

y and m, independently, are integers equal to 0, 1, 2 or 3, provided that y plus m equals 3;

$R^1$ is hydrogen, $C_1$-$C_4$ alkyl, phenyl, thienyl, amino or $C_1$-$C_4$ alkanoylamino;

X is O, S or N-$R^2$, where $R^2$ is hydrogen or $C_1$-$C_4$ alkyl; or a pharmaceutically-acceptable salt or biologically-cleavable ester thereof, which comprises

(a) condensing a compound of Formula (2):

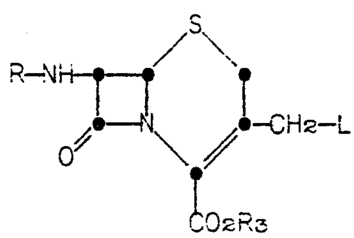

(2)

in which L is a leaving group, $R_3$ is hydrogen or a carboxy-protecting group, and R is as defined in claim 1 or 2; with a bicyclicpyridine compound of the Formula:

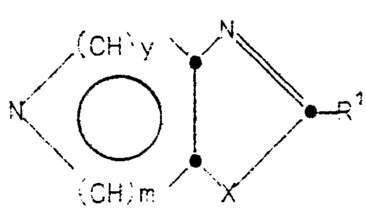

,

and if desired, removing any protecting group which may be present, and/or salifying or esterifying the product.

(b) acylating a compound of Formula (1) in which R is hydrogen, or a salt or 4'-ester thereof, with an acid of the Formula:

$$R'-\underset{\underset{N-OR''}{\|}}{C}-COOH$$

or an activated derivative thereof, and if desired, removing any protecting group present and/or salifying or esterifying the product.

(c) deacylating a compound of Formula (1) in which R is other than hydrogen, or a salt or ester thereof to form a compound in which R is hydrogen, or a salt or ester thereof.

2. A process for preparing a compound of Formula (1):

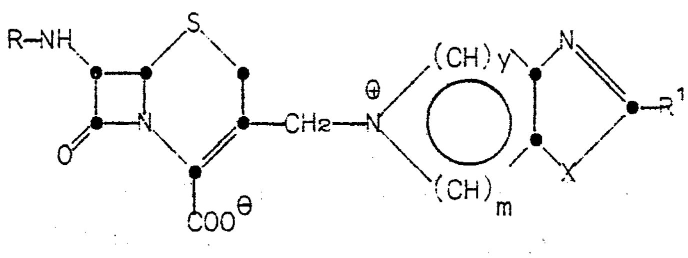

1

in which R is hydrogen, formyl, α-aminoadipoyl, protected α-aminoadipoyl, or an acyl group of the formula

$$R'-\underset{\underset{N\diagdown O-R''}{\|}}{C}-\overset{\overset{O}{\|}}{C}-$$

in which R' is a 5- or 6-membered heterocyclic ring of the formulae

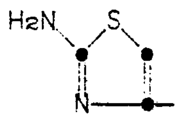, 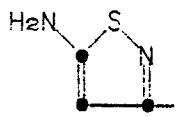, 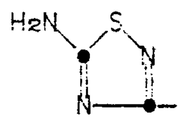, 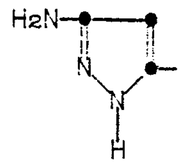,

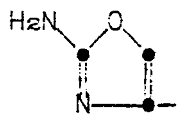, 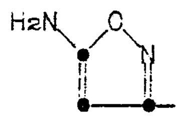, 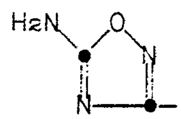, 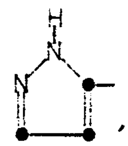,

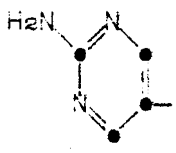, 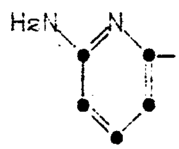, or 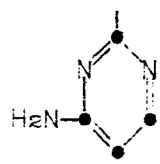;

R'' is hydrogen, $C_1$-$C_4$ alkyl, a carboxy-substituted alkyl or carboxy-substituted cycloalkyl group of the formula:

$$-\overset{a}{\underset{b}{C}}-(CH_2)_n-COR'''$$

in which n is 0-3; a and b when taken separately are, independently, hydrogen or $C_1$-$C_3$ alkyl, and when taken together with the carbon to which they are bonded form a $C_3$-$C_7$ carbocyclic ring; R''' is hydroxy, $C_1$-$C_4$ alkoxy, amino, or OR°, in which R° is indanyl, phthalidyl, or an acyloxymethyl group of the formula $-CH_2-O-C(O)-R_2$ in which $R_2$ is $C_1$-$C_4$ alkyl or phenyl; or COOR° is a protected carboxy group;

or R'' is an N-substituted carbamoyl group of the formula

$$-\overset{O}{\overset{\|}{C}}-NHR''''$$

in which R'''' is $C_1$-$C_4$ alkyl, phenyl or $C_1$-$C_3$ alkyl substituted by phenyl;

y and m, independently, are integers equal to 0, 1, 2 or 3, provided that y plus m equals 3;

$R^1$ is hydrogen, $C_1$-$C_4$ alkyl, phenyl, thienyl, amino or $C_1$-$C_4$ alkanoylamino;

X is O, S or N-$R^2$, where $R^2$ is hydrogen or $C_1$-$C_4$ alkyl; or a pharmaceutically-acceptable salt or biologically-cleavable ester thereof, provided that when R' is

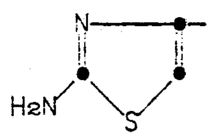 ;

and R'' is $C_1$-$C_4$ alkyl, or if n=0, and a and b, independently, are hydrogen, methyl, ethyl or when a and b are taken together with the carbon to which they are attached form a $C_3$-$C_5$ carbocyclic ring; and y=1 and m=2; and X=S, then $R^1$ may only be phenyl, thienyl or $C_1$-$C_4$ alkanoylamino, said process comprising

(a) condensing a compound of Formula (2):

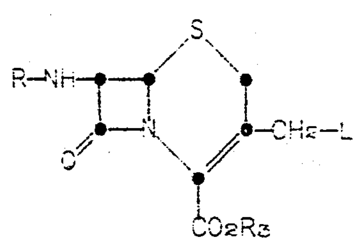 (2)

in which L is a leaving group, $R_3$ is hydrogen or a carboxy-protecting group, and R is as defined in claim 1 or 2; with a bicyclicpyridine compound of the Formula:

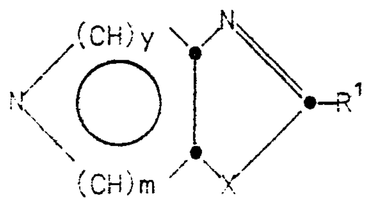

,

and if desired, removing any protecting group which may be present, and/or salifying or esterifying the product.

(b) acylating a compound of Formula (1) in which R is hydrogen, or a salt or 4'-ester thereof, with an acid of the Formula:

R'-C-COOH
   ‖
   N-OR''

or an activated derivative thereof, and if desired, removing any protecting group present and/or salifying or esterifying the product.

(c) deacylating a compound of Formula (1) in which R is other than hydrogen, or a salt or ester thereof to form a compound in which R is hydrogen, or a salt or ester thereof.

3. A process for preparing a compound of Formula (1), or a pharmaceutically-acceptable salt or ester thereof, as claimed in claim 1 or 2 in which R' is 2-amino-thiazol-4-yl.

4. A process for preparing a compound of Formula (1), or a pharmaceutically-acceptable salt or ester thereof, as claimed in claim 3 in which y is 1 and m is 2.

5. A process for preparing a compound of Formula (1), as claimed in any one of claims 1 to 4 in which X is N-$R^2$ or O.

6. A process for preparing a compound of Formula (1), as claimed in any one of claims 1 to 4 in which X is S.

7. A process as claimed in any one of claims 1 to 6 for preparing

syn-7-[2-(2-Aminothiazol-4-yl)-2-(2-carboxyprop-2-yl)oxyiminoacetamido]-3-(1H-imidazolo[4,5-c]-pyridinium-5-ylmethyl)-3-cephem-4-carboxylate

syn-7-[2-(2-Aminothiazol-4-yl)-2-methoxyiminoacetamido]-3-(1H-imidazolo]4,5-c]pyridinium-5-ylmethyl)-3-cephem-4-carboxylate

syn-7-[2-(2-Aminothiazol-4-yl)-2-methoxyiminoacetamido]-3-(3-methyl-3H-imidazolo[4,5-c]pyridinium-5-ylmethyl)-3-cephem-4-carboxylate

syn-7-[2-(2-Aminothiazol-4-yl)-2-methoxyiminoacetamido]-3-(1-H-imidazolo[4,5-c]pyridinium-5-ylmethyl)-3-cephem-4-carboxylate

syn-7-[2-(2-Aminothiazol-4-yl)-2-methoxyiminoacetamido]-3-(2-methyl-1H-imidazolo[4,5-c]pyridinium-5-ylmethyl)-3-cephem-4-carboxylate

syn-7-[2-(2-Aminothiazol-4-yl)-2-methoxyiminoacetamido]-3-(1,2-dimethyl-1H-imidazolo[4,5-c]pyridinium-5-ylmethyl)-3-cephem-4-carboxylate

syn-7-[2-(2-Aminothiazole-4-yl)-2-methoxyiminoacetamido]-3-(2,3-dimethyl-3H-imidazolo[4,5-c]-pyridinium-5-ylmethyl)-3-cephem-4-carboxylate

syn-7-[2-(2-Aminothiazol-4-yl)-2-methoxyiminoacetamido]-3-[2-(2-thienyl)-1H-imidazolo[4,5-c]-pyridinium-5-ylmethyl)-3-cephem-4-carboxylate

syn-7-[2-(2-Aminothiazole-4-yl)-2-methoxyiminoacetamido]-3-(2-phenyl-1H-imidazolo[4,5-b]-pyridinium-5-ylmethyl)-3-cephem-4-carboxylate

syn-7-[2-(2-Aminothiazol-4-yl)-2-methoxyiminoacetamido]-3-(2-aminooxazolo[4,5-b]-pyridinium-4-ylmethyl)-3-cephem-4-carboxylate

syn-7-[2-(2-Aminothiazol-4-yl)-2-methoxyiminoacetamido]-3-(2-methyloxazolo[4,5-c]-pyridinium-5-ylmethyl)-3-cephem-4-carboxylate or

syn-7-[2-(2-Aminothiazol-4-yl)-2-methoxyiminoacetamido]-3-(2-aminothiazolo[5,4-b]-pyridinium-4-ylmethyl)-3-cephem-4-carboxylate
or a pharmaceutically-acceptable salt or biologically-cleavable ester thereof.

8. A process as claimed in any one of claims 1 to 6 for preparing

syn-7-[2-(2-Aminothiazol-4-yl)-2-methoxyiminoacetamido]-3-(thiazolo[4,5-c]-pyridinium-5-ylmethyl)-3-cephem-4-carboxylate

syn-7-[2-(2-Aminothiazol-4-yl)-2-methoxyiminoacetamido]-3-(2-methylthiazolo[4,5-c]pyridinium-5-ylmethyl)-3-cephem-4-carboxylate or

syn-7-[2-(2-aminothiazolo-4-yl)-2-methoxyiminoacetamido]-3-(2-methylthiazolo[5,4-c]pyridinium-5-ylmethyl)-3-cephem-4-carboxylate.

syn-7-[2-(2-Aminothiazol-4-yl)-2-methoxyiminoacetamido]-3-(2-ethylthiazolo[4,5-c]pyridinium-5-ylmethyl)-3-cephem-4-carboxylate or a pharmaceutically-acceptable salt or biologically-cleavable ester thereof.

9. A compound of Formula (1), or a biologically-cleavable ester or pharmaceutically-acceptable salt thereof, whenever prepared according to a process as claimed in any one of claims 1 to 8.